(19) Europäisches Patentamt
European Patent Office
Office européen des brevets

(11) Publication number: **0 445 304 A1**

# (12) EUROPEAN PATENT APPLICATION published in accordance with Art. 158(3) EPC

(21) Application number: **90913883.6**

(22) Date of filing: **25.09.90**

(86) International application number:
**PCT/JP90/01234**

(87) International publication number:
**WO 91/04276 (04.04.91 91/08)**

(51) Int. Cl.5: **C07K 15/14**, C12P 21/00, A61K 37/02, C12N 15/12, C12R 1/91

(30) Priority: **25.09.89 JP 246270/89**

(43) Date of publication of application:
**11.09.91 Bulletin 91/37**

(84) Designated Contracting States:
**AT BE CH DE FR GB IT LI LU NL SE**

(71) Applicant: **Asahi Kasei Kogyo Kabushiki Kaisha**
**2-6, Dojimahama 1-chome Kita-ku**
**Osaka-shi Osaka 530(JP)**

(72) Inventor: **YAMAMOTO, Shuji**
**168-27, Obuchi**
**Fuji-shi Shizuoka-ken 417(JP)**
Inventor: **GOMI, Komakazu**
**2-19-10, Senpukugaoka**
**Susono-shi Shizuoka-ken 410-11(JP)**
Inventor: **OGAWA, Kohei**
**B-23 Endo Apartment 68, Aoshima-cho**
**Fuji-shi Shizuoka-ken 417(JP)**

(74) Representative: **Boeters, Hans Dietrich, Dr. et al**
**Boeters & Bauer Bereiteranger 15**
**W-8000 München 90(DE)**

## (54) ISOLATED, PHYSIOLOGICALLY ACTIVE HUMAN THROMBOMODULIN POLYPEPTIDE.

(57) An isolated polypeptide (type II polypeptide) comprising a physiologically active human thrombomodulin polypeptide which can promote the activation of protein C by thrombin, wherein the peptide chain has a sugar chain containing chondroitin and/or chondroitin sulfate. It can be isolated from a conventional culture medium containing, for example, a product of genetic expression of a human thrombomodulin polypeptide by recombinant DNA technology, and is excellent in anticoagulant, platelet agglutination inhibiting and thrombolytic activities.

EP 0 445 304 A1

## Technical Field

The present invention relates to a novel thrombomodulin. More particularly, the present invention is concerned with an isolated physiologically active human thrombomodulin polypeptide which has a sugar chain comprising chondroitin and/or chondroitin sulfate attached to the peptide chain of the polypeptide. The thrombomodulin polypeptide of the present invention participates in anticoagulation of blood and fibrinolysis with respect to blood coagulation, and has excellent activities of anticoagulation, platelet aggregation inhibition and thrombolysis as compared to the conventionally known human thrombomodulin polypeptide. The polypeptide of the present invention is therefore useful as an active ingredient of drugs, particularly those for the treatment of diseases involving undesired blood-coagulation. The present invention also relates to a process for preparing this novel human thrombomodulin polypeptide and to a pharmaceutical composition comprising this polypeptide as an active ingredient.

In the present specification, amino acids and peptides are represented using abbreviations approved by the IUPAC-IUB Commission on Biochemical Nomenclature (CBN). Amino acids and the like having optical isomers are of the L-configuration unless otherwise specified. Further, unless otherwise specified, the left end and right end of the amino acid sequences of peptides are the N-terminus and C-terminus, respectively.

Further, unless otherwise specified, the left end and right end of the sequence of deoxyribonucleotides are the 5' end and 3' end, respectively.

## Background Art

Protein C is known as a protein which is dependent on vitamin K and which plays an important role in the blood coagulation mechanism. In recent years, it has been reported that a substance which binds to thrombin to thereby accelerate the thrombin-catalyzed activation of protein C and repress platelet activation and fibrin formation by the action of thrombin, is present in rabbit lung, bovine lung, human lung, human placenta and the like. Such a substance is generally called "thrombomodulin".

With respect to the substance present in rabbit lung, reference may be made to, for example, C. T. Esmon et al. [Proc. Natl. Acad. Sci. U.S.A., Vol. 78, pp. 2249-2252 (1981) and J. Biol. Chem. Vol. 257, pp. 7944-7947 (1982)] and N. L. Esmon et al. [J. Biol. Chem., Vol. 257, pp. 859-864 (1982); and J. Biol. Chem. Vol. 258, pp. 12238-12242 (1982)].

Further, with respect to the substance present in human placenta, reference may be made to, for example, H. H. Salem et al. [J. Biol. Chem. Vol. 259, pp. 12246-12251 (1984)]. Moreover, with respect to a similar substance present in human lung, reference may be made to, for example, Kusumoto et al. [Biochemistry, Japan, Vol. 57, p. 1102 (1985)].

Meanwhile, S. Yamamoto, who is one of the present inventors, and colleagues have disclosed the cloning of a human-derived thrombomodulin cDNA [International Application Publication No. W088/05033 (International Application No. PCT/JP88/ 00011)]. In making the invention described in the specification of the above International Application Publication, Yamamoto et al. succeeded in obtaining a DNA coding for human thrombomodulin. A microorganism or cell was transformed with a replicable recombinant DNA containing the above-mentioned DNA to obtain transformants. The transformants were selected from parent cells and then cultured to obtain a culture which contained a substance produced by expressing the DNA. The culture was then subjected twice to column chromatography, which is one of the known purification treatments, to obtain a purified thorombomodulin.

In the course of studying the properties of this expression product, the present inventors have unexpectedly found that the thrombomodulin, which is a glycoprotein, has heterogeneity in its sugar chain. The present inventors have made further extensive and intensive studies on the purification of the culture containing the expression product. As a result, they have surprisingly found that the thrombomodulin can be separated into two different physiologically active thrombomodulin polypeptides by subjecting the culture to purification in which a separating treatment is conducted utilizing a difference in a property selected from isoelectric point, molecular weight and reactivity to anti-chondroitin antibody or anti-chondroitin sulfate antibody. For example, the culture was separated by means of an anion-exchange column into two fractions, that is, a fraction having a peak of activity in a region of high ionic strength elution and a fraction having a peak of activity in a region of low ionic strength elution. Each fraction was then purified to isolate two types of thrombomodulin polypeptides. The polypeptide obtained by purifying the eluted fraction having a peak of activity in the low ionic strength elution region was found to be identical to the thrombomodulin polypeptide (hereinafter frequently referred to as "type I thrombomodulin polypeptide") obtained by the purification process described in Examples 17-21 of the above-mentioned International Application Publication No. W088/05033. On the other hand, the polypeptide obtained by purifying the eluted fraction having a peak of activity in the high ionic strength elution region was found to have the same amino acid sequence as that of the type I thrombomodulin polypeptide, but have a higher molecular weight and a lower isoelectric point than the type I thrombomodulin polypeptide.

Further, the sugar chain portion was analyzed with respect to each of the two types of thrombomodulin polypeptides. As a result, it has been found that the polypeptide obtained by purifying the fraction having a peak of activity in the high ionic strength elution region has a sugar chain comprising chondroitin and/or chondroitin sulfate attached thereto (hereinafter frequently referred to as "type II thrombomodulin polypeptide"), and that the type I thrombomodulin polypeptide has substantially no sugar chain comprising chondroitin and/or chondroitin sulfate. Further, it has been found that the type II thrombomodulin polypeptide is a novel thrombomodulin which exhibits a physiological activity differing from that of the type I thrombomodulin polypeptide due to the presence of chondroitin and/or chondroitin sulfate attached thereto, and that the type II thrombomodulin polypeptide has high physiological activities as compared to those of the type I thrombomodulin polypeptide. The present invention has been completed based on these novel findings.

In the present invention, the type I thrombomodulin polypeptide and the type II thrombomodulin polypeptide are hereinafter frequently referred to simply as "type I polypeptide" and "type II polypeptide", respectively.

## Disclosure of the Present Invention

Accordingly, an object of the present invention is to provide an isolated physiologically active human thrombomodulin polypeptide (type II polypeptide) which has a sugar chain comprising chondroitin and/or chondroitin sulfate attached to the peptide chain of the polypeptide.

Another object of the present invention is to provide a process for preparing the type II polypeptide.

Still another object of the present invention is to provide a pharmaceutical composition containing, as an active ingredient, the above-mentioned type II polypeptide.

The foregoing and other objects, features and advantages of the present invention will be apparent from the following detailed description and appended claims taken in connection with the accompanying drawings.

In one aspect of the present invention, there is provided an isolated physiologically active human thrombomodulin polypeptide which has the activity of promoting the thrombin-catalyzed activation of protein C and which has a sugar chain comprising chondroitin and/or chondroitin sulfate attached to the peptide chain of the polypeptide.

In another aspect of the present invention, there is provided a process for preparing an isolated physiologically active human thrombomodulin polypeptide which has the activity of promoting the thrombin-catalyzed activation of protein C and which has a sugar chain comprising chondroitin and/or chondroitin sulfate attached to the peptide chain of the polypeptide, which comprises:

(1) providing a human thrombomodulin composition comprising a type I thrombomodulin polypeptide and a type II thrombomodulin polypeptide,

the type I thrombomodulin polypeptide being defined as a physiologically active human thrombomodulin polypeptide which has the activity of promoting the thrombin-catalyzed activation of protein C and which is substantially free of a sugar chain comprising chondroitin and/or chondroitin sulfate attached to the peptide chain of the polypeptide, and

the type II thrombomodulin polypeptide being defined as a physiologically active human thrombomodulin polypeptide which has the activity of promoting the thrombin-catalyzed activation of protein C and which has a sugar chain comprising chondroitin and/or chondroitin sulfate attached to the peptide chain of the polypeptide;

(2) subjecting the human thrombomodulin composition to a separating treatment capable of separating the type I and type II thrombomodulin polypeptides from each other due to a difference in a property selected from isoelectric point, molecular weight and reactivity to an anti-chondroitin antibody and/or an anti-chondroitin sulfate antibody between the type I and the type II thrombomodulin polypeptides, thereby separating the type I thrombomodulin polypeptide and the type II thrombomodulin polypeptide from each other; and

(3) recovering the type II thrombomodulin polypeptide.

In still another aspect of the present invention, there is provided a pharmaceutical composition comprising an effective amount of an isolated physiologically active human thrombomodulin polypeptide which has the activity of promoting the thrombin-catalyzed activation of protein C and which has a sugar chain comprising chondroitin and/or chondroitin sulfate attached to the peptide chain of the polypeptide, and at least one pharmaceutically acceptable carrier, diluent or excipient.

The structure of the amino acid portion of the type II polypeptide of the present invention is only required to comprise at least a site that is necessary to exhibit the desired activity (that is, the activity of promoting the thrombin-catalyzed activation of protein C). For example, it is known that the polypeptide comprising the 366th to 480th 115 amino acids of the sequence of 575 amino acids (shown in Fig. 1) inclusive of the signal polypeptide portion of the thrombomodulin, the signal polypeptide portion being represented by the formula:

Met Leu Gly Val Leu Val Leu Gly Ala Leu Ala Leu
Ala Gly Leu Gly Phe Pro,

has the desired activity [M. Sushi et al., J. Biol. Chem., Vol. 264, pp. 10351-10353 (1989)]. Further, the type II polypeptide is characterized by having a sugar chain comprising chondroitin and/or chondroitin sulfate attached to at least one of the sites of the peptide chain, to which sites, a sugar chain comprising chondroitin and/or chondroitin sulfate can be attached, the peptide chain comprising at least a site that is necessary to exhibit the desired activity (that is, the activity of promoting the thrombin-catalyzed activation of protein C).

The chondroitin and/or chondroitin sulfate contained in the sugar chain attached to the type II polypeptide of the present invention is glycosaminoglycan, namely, a polysaccharide which comprises a structure of recurrence of a disaccharide unit comprising uronic acid and non-sulfonated and/or sulfonated N-acetylgalactosamine.

According to M. Isemura et al. [Biochem. Biophys. Acta. Vol. 411, pp. 11-21 (1975)], M. A. Bourdon et al. [Proc. Natl. Acad. Sci. U.S.A., Vol. 82, pp. 1321-1325 (1985), and C. H. Pearson et al. [J. Biol. Chem. Vol. 258, pp. 15101-15104 (1985)], a site of the polypeptide to which a sugar chain comprising chondroitin and/or chondroitin sulfate can be attached, is present at the Ser-Gly sequence portion. Therefore, the sites of full-length thrombomodulin to which a sugar chain comprising chondroitin and/or chondroitin sulfate can be attached, are the 452nd, 482nd, 490th, 492nd and 515th positions of the amino acid sequence shown in Fig. 1. Thus, as an amino acid sequence to which a sugar chain can be attached, there may be mentioned, for example, a polypeptide comprising the 366th to 480th 115 amino acids, a polypeptide comprising the 481st to 516th 36 amino acids of the amino acid sequence shown in Fig. 1, and a polypeptide comprising 151 amino acids having these amino acid sequences as its N-terminal region and C-terminal region, respectively.

Accordingly, as the peptide chain of the type II polypeptide of the present invention, that is, a peptide chain which has both an activity exhibiting site and a site to which a sugar chain comprising chondroitin and/or chondroitin sulfate can be attached, there may be mentioned, for example, an amino acid sequence of the 366th to 480th 115 amino acids shown in Fig. 1, represented by the formula (I):

Val Asp Pro Cys Phe Arg Ala Asn Cys
Glu Tyr Gln Cys Gln Pro Leu Asn Gln
Thr Ser Tyr Leu Cys Val Cys Ala Glu
Gly Phe Ala Pro Ile Pro His Glu Pro

His Arg Cys Gln Met Phe Cys Asn Gln
Thr Ala Cys Pro Ala Asp Cys Asp Pro
Asn Thr Gln Ala Ser Cys Glu Cys Pro
Glu Gly Tyr Ile Leu Asp Asp Gly Phe
Ile Cys Thr Asp Ile Asp Glu Cys Glu
Asn Gly Gly Phe Cys Ser Gly Val Cys
His Asn Leu Pro Gly Thr Phe Glu Cys
Ile Cys Gly Pro Asp Ser Ala Leu Val
Arg His Ile Gly Thr Asp Cys ... (I)

and an amino acid sequence of 151 amino acids which comprises the amino acid sequence of the formula (I) and, attached thereto at its C-terminus, an amino acid sequence of 36 amino acids represented by the formula (II):

Asp Ser Gly Lys Val Asp Gly Gly Asp Ser
Gly Ser Gly Glu Pro Pro Pro Ser Pro Thr
Pro Gly Ser Thr Leu Thr Pro Pro Ala Val
Gly Leu Val His Ser Gly ... (II).

Further, the amino acid structure of the type II polypeptide of the present invention may comprise, for example, an amino acid sequence of 498 amino acids which is obtained by removing the signal polypeptide portion, the membrane-binding site and the cytoplasm site (that is, the 517th to 575th amino acids in Fig. 1) from the entire amino acid sequence (Fig. 1). This amino acid sequence of 498 amino acids is an amino acid sequence comprising the amino acid sequence of formula (I) and, attached thereto at its N-terminus and C-terminus, an amino acid sequence represented by the formula (III):

Ala Pro Ala Glu Pro Gln Pro Gly Gly Ser
Gln Cys Val Glu His Asp Cys Phe Ala Leu
Tyr Pro Gly Pro Ala Thr Phe Leu Asn Ala
Ser Gln Ile Cys Asp Gly Leu Arg Gly His
Leu Met Thr Val Arg Ser Ser Val Ala Ala
Asp Val Ile Ser Leu Leu Leu Asn Gly Asp
Gly Gly Val Gly Arg Arg Arg Leu Trp Ile
Gly Leu Gln Leu Pro Pro Gly Cys Gly Asp
Pro Lys Arg Leu Gly Pro Leu Arg Gly Phe
Gln Trp Val Thr Gly Asp Asn Asn Thr Ser
Tyr Ser Arg Trp Ala Arg Leu Asp Leu Asn
Gly Ala Pro Leu Cys Gly Pro Leu Cys Val
Ala Val Ser Ala Ala Glu Ala Thr Val Pro
Ser Glu Pro Ile Trp Glu Glu Gln Gln Cys
Glu Val Lys Ala Asp Gly Phe Leu Cys Glu
Phe His Phe Pro Ala Thr Cys Arg Pro Leu
Ala Val Glu Pro Gly Ala Ala Ala Ala Ala
Val Ser Ile Thr Tyr Gly Thr Pro Phe Ala
Ala Arg Gly Ala Asp Phe Gln Ala Leu Pro
Val Gly Ser Ser Ala Ala Val Ala Pro Leu

Gly Leu Gln Leu Met Cys Thr Ala Pro Pro
Gly Ala Val Gln Gly His Trp Ala Arg Glu
Ala Pro Gly Ala Trp Asp Cys Ser Val Glu
Asn Gly Gly Cys Glu His Ala Cys Asn Ala
Ile Pro Gly Ala Pro Arg Cys Gln Cys Pro
Ala Gly Ala Ala Leu Gln Ala Asp Gly Arg
Ser Cys Thr Ala Ser Ala Thr Gln Ser Cys
Asn Asp Leu Cys Glu His Phe Cys Val Pro
Asn Pro Asp Gln Pro Gly Ser Tyr Ser Cys
Met Cys Glu Thr Gly Tyr Arg Leu Ala Ala
Asp Gln His Arg Cys Glu Asp Val Asp Asp
Cys Ile Leu Glu Pro Ser Pro Cys Pro Gln
Arg Cys Val Asn Thr Gln Gly Gly Phe Glu
Cys His Cys Tyr Pro Asn Tyr Asp Leu Val
Asp Gly Glu Cys Val Glu Pro ... (III),

and an amino acid sequence represented by the formula (II):

Asp Ser Gly Lys Val Asp Gly Gly Asp Ser
Gly Ser Gly Glu Pro Pro Pro Ser Pro Thr
Pro Gly Ser Thr Leu Thr Pro Pro Ala Val
Gly Leu Val His Ser Gly ... (II),

respectively.

Another example of the amino acid structure of the type II polypeptide of the present invention may comprise the following amino acid sequence of 557 amino acids of thrombomodulin in mature form, which is obtained by removing the signal polypeptide portion from the entire amino acid sequence (Fig. 1):

Ala Pro Ala Glu Pro Gln Pro Gly Gly Ser
Gln Cys Val Glu His Asp Cys Phe Ala Leu
Tyr Pro Gly Pro Ala Thr Phe Leu Asn Ala
Ser Gln Ile Cys Asp Gly Leu Arg Gly His
Leu Met Thr Val Arg Ser Ser Val Ala Ala
Asp Val Ile Ser Leu Leu Leu Asn Gly Asp
Gly Gly Val Gly Arg Arg Arg Leu Trp Ile
Gly Leu Gln Leu Pro Pro Gly Cys Gly Asp
Pro Lys Arg Leu Gly Pro Leu Arg Gly Phe
Gln Trp Val Thr Gly Asp Asn Asn Thr Ser
Tyr Ser Arg Trp Ala Arg Leu Asp Leu Asn
Gly Ala Pro Leu Cys Gly Pro Leu Cys Val
Ala Val Ser Ala Ala Glu Ala Thr Val Pro
Ser Glu Pro Ile Trp Glu Glu Gln Gln Cys
Glu Val Lys Ala Asp Gly Phe Leu Cys Glu
Phe His Phe Pro Ala Thr Cys Arg Pro Leu
Ala Val Glu Pro Gly Ala Ala Ala Ala Ala
Val Ser Ile Thr Tyr Gly Thr Pro Phe Ala
Ala Arg Gly Ala Asp Phe Gln Ala Leu Pro
Val Gly Ser Ser Ala Ala Val Ala Pro Leu
Gly Leu Gln Leu Met Cys Thr Ala Pro Pro
Gly Ala Val Gln Gly His Trp Ala Arg Glu
Ala Pro Gly Ala Trp Asp Cys Ser Val Glu

Asn Gly Gly Cys Glu His Ala Cys Asn Ala
Ile Pro Gly Ala Pro Arg Cys Gln Cys Pro
Ala Gly Ala Ala Leu Gln Ala Asp Gly Arg
Ser Cys Thr Ala Ser Ala Thr Gln Ser Cys
Asn Asp Leu Cys Glu His Phe Cys Val Pro
Asn Pro Asp Gln Pro Gly Ser Tyr Ser Cys
Met Cys Glu Thr Gly Tyr Arg Leu Ala Ala
Asp Gln His Arg Cys Glu Asp Val Asp Asp
Cys Ile Leu Glu Pro Ser Pro Cys Pro Gln
Arg Cys Val Asn Thr Gln Gly Gly Phe Glu
Cys His Cys Tyr Pro Asn Tyr Asp Leu Val
Asp Gly Glu Cys Val Glu Pro Val Asp Pro
Cys Phe Arg Ala Asn Cys Glu Tyr Gln Cys
Gln Pro Leu Asn Gln Thr Ser Tyr Leu Cys
Val Cys Ala Glu Gly Phe Ala Pro Ile Pro
His Glu Pro His Arg Cys Gln Met Phe Cys
Asn Gln Thr Ala Cys Pro Ala Asp Cys Asp
Pro Asn Thr Gln Ala Ser Cys Glu Cys Pro
Glu Gly Tyr Ile Leu Asp Asp Gly Phe Ile
Cys Thr Asp Ile Asp Glu Cys Glu Asn Gly
Gly Phe Cys Ser Gly Val Cys His Asn Leu
Pro Gly Thr Phe Glu Cys Ile Cys Gly Pro
Asp Ser Ala Leu Val Arg His Ile Gly Thr
Asp Cys Asp Ser Gly Lys Val Asp Gly Gly
Asp Ser Gly Ser Gly Glu Pro Pro Pro Ser

Pro Thr Pro Gly Ser Thr Leu Thr Pro Pro
Ala Val Gly Leu Val His Ser Gly Leu Leu
Ile Gly Ile Ser Ile Ala Ser Leu Cys Leu
Val Val Ala Leu Leu Ala Leu Leu Cys His
Leu Arg Lys Lys Gln Gly Ala Ala Arg Ala
Lys Met Glu Tyr Lys Cys Ala Ala Pro Ser
Lys Glu Val Val Leu Gln His Val Arg Thr
Glu Arg Thr Pro Gln Arg Leu.

The polypeptide of the present invention may also contain, as its N-terminal amino acid sequence, at least a part of the signal polypeptide sequence, for example, the Phe-Pro sequence and the like.

The amino acid structure of the polypeptide can be partially changed by artificial mutation without causing a significant change of the activity of the polypeptide. Further, it is known that a thrombomodulin exists in nature, whose amino acid sequence is partially changed. More specifically, a homologous variant of thrombomodulin is known, which homologous variant has Ala in place of Val at the 473rd position of the entire amino acid sequence shown in Fig. 1, the entire amino acid sequence having been determined by S. Yamamoto who is one of the present inventors, and his colleagues [D. Wen et al., Biochemistry, Vol. 26, pp. 4350-4357 (1987)]. In the case of the type II polypeptide of the present invention, a homologous variant thereof wherein the 473rd position of the amino acid sequence shown in Fig. 1 is Ala in place of Val, exhibits an activity equal to the activity of the type II polypeptide. Therefore, examples of the type II polypeptide of the present invention include polypeptides which have amino acid sequences corresponding to homologous variants of the polypeptides having the above-mentioned various amino acid sequences.

The amount of the sugar chain comprising chondroitin and/or chondroitin sulfate attached to type II polypeptide of the present invention, is 5 to 2000 µg, preferably 10 to 1000 µg, more preferably 30 to 500 µg in terms of the amount of uronic acid per milligram of the amino acid portion of the polypeptide.

The process for preparing type II polypeptide of the present invention comprises (1) providing a human thrombomodulin composition comprising a type I polypeptide and a type II polypeptide, (2) subjecting the human thrombomodulin composition to a separating treatment capable of separating the type I polypeptide and the type II polypeptide from each other due to a difference in a property selected from isoelectric point, molecular weight and reactivity (binding properties) to an anti-chondroitin antibody and/or an anti-chondroitin sulfate antibody between the type I polypeptide and type II polypeptide, thereby separating the type I polypeptide and the type II polypeptide from each other, and (3) recovering the separated type II polypeptide.

As a composition comprising a type I polypeptide and a type II polypeptide, there may be mentioned, for example, an extract or culture of cells established by introducing an expression vector having a cDNA coding for the polypeptide to the cells. The above-mentioned separating step (2) is incorporated into the purification procedure for the culture as one step thereof. Except for the separating treatment the purification procedure can be conducted using standard purification techniques, for example, various chromatography techniques individually or in combination.

Examples of methods for separating the type I polypeptide and the type II polypeptide from each other by utilizing a difference in properties therebetween include a method wherein the difference in isoelectric point due to the presence of the sugar chain comprising chondroitin and/or chondroitin sulfate attached to the polypeptide is utilized and the separating treatment is conducted by ion-exchange chromatography or electrophoresis; a method wherein the difference in molecular weight of the polypeptide due to the presence of the sugar chain comprising chondroitin and/or chondroitin sulfate attached to the polypeptide is utilized and the separating treatment is conducted by gel filtration or electrophoresis; and a method wherein the difference in reactivity (binding properties) to an anti-chondroitin antibody and/or anti-chondroitin sulfate antibody recognizing the sugar chain comprising chondroitin and/or chondroitin sulfate is utilized and the separating treatment is conducted by affinity chromatography.

An illustrative example of production is explained below. An expression vector comprising a DNA coding for a polypeptide which comprises the 1st to 516th 516 amino acids shown in Fig. 1 is transfected into animal cells to establish cells for producing the polypeptide. The established cells are then cultured, and the resultant culture is adsorbed onto a carrier for anion-exchange chromatography and eluted with a linear concentration gradient of sodium chloride to obtain two activity peaks. Fractions of each activity peak (for example, fraction Nos. 8 to 18 and fraction Nos. 38 to 58 in Fig. 3) are collected. These fractions are desalted, and then purified by subjecting to chromatography using a diisopropylphosphate-thrombin-Sepharose column and then to gel filtration. When only the activity peak fractions eluted at a higher concentration of NaCl (fraction Nos. 38 to 58 in Fig. 3: NaCl concentration, around 0.65M) are collected and purified, the obtained product is an isolated type II polypeptide having a sugar chain comprising chondroitin and/or chondroitin sulfate attached thereto. When only the activity peak fractions eluted at a lower concentration of NaCl (fraction Nos. 8 to 18 in Fig. 3: NaCl concentration, around 0.3M) are collected and purified, the obtained product is an isolated type I polypeptide having no sugar chain comprising chondroitin and/or chondroitin sulfate (which corresponds to the product obtained in the Examples of International Application Publication No. W088/05033 by S. Yamamoto et al.). These polypeptide products are substantially pure and free from each other.

Further, type II polypeptide is higher than type I polypeptide with respect to various activities represented by promotion of the thrombin-catalyzed activation of protein C, reduction of the thrombin clotting time and the activated partial thromboplastin time, inhibition of platelet aggregation, and suppression of the thrombin-caused activation of factor V. The difference in these activities is considered to be caused by the presence of chondroitin and/or chondroitin tached to the peptide chain of the type II polypeptide.

As mentioned above, the present invention has been completed based on the findings that a culture containing a polypeptide as an expression product (that is, human thrombomodulin) having the activity of promoting the thrombin-catalyzed activation of protein C described in the above-mentioned International Application Publication No. W088/05033 unexpectedly comprises two different thrombomodulin polypeptides, that is, type II thrombomodulin polypeptide and type I thrombomodulin polypeptide, and that the type II polypeptide, which is a novel thrombomodulin, surprisingly has a physiological activities higher than those of the conventional type I polypeptide, due to the presence of a sugar chain comprising chondroitin and/or chondroitin sulfate.

By the present invention, there is for the first time provided a novel thrombomodulin having higher physiological activities, namely type II polypeptide, which can be used for medical purposes and the like more effectively than the conventional thrombomodulin.

Illustratively stated, the human thrombomodulin polypeptide of the present invention which has a sugar chain comprising chondroitin and/or chondroitin sulfate has excellent anticoagulant, platelet aggregation inhibiting and thrombolytic activities. Hence, the thrombomodulin polypeptide can be advantageously used, for example, for the treatment and prevention of diseases, such as myocardial infarction, thrombosis, embolism, obstruction of peripheral blood vessels, arteriosclerosis obliterans, disseminated intravascular coagulation (DIC) syndrome, angina pectoris, transient ischemic attack, toxemia of pregnancy. Especially when such diseases are in a state where it is required to exhibit higher physiological activities in a short period, for example, when the diseases are in an acute phase, more effective medical treatment can be provided by the use of the type II thrombomodulin polypeptide of the present invention having high activities.

When the type II polypeptide of the present invention is used for the treatment of the above-mentioned diseases, the type II polypeptide may be used in the form of a mixture thereof with at least one pharmaceutically acceptable carrier, diluent or excipient. That is, an effective amount of the type II polypeptide of the present invention for treating or preventing the above-mentioned diseases can be mixed with an appropriate amount of a carrier, a diluent or an excipient to prepare a pharmaceutical composition which is suitable for effective administration to a patient. In particular, when the polypeptide of the present invention is used in the form of an injection, there may be employed as an additive, a thickening agent such as sucrose, glycerol, methylcellulose or carboxymethylcellulose, a pH adjusting agent comprising various inorganic salts, and the like.

The dose of the physiologically active polypeptide of the present invention per adult varies with the age, sex, weight, conditions, etc., of the patient. In general, however, the dose is about 0.1 to about 200 mg. The present polypeptide may be administered once a day or, if desired, several times a day.

## Best Mode for Carrying Out the Invention

The present invention will be described in more detail with reference to the following Examples and Preparation Examples, which should not be construed as limiting the scope of the present invention.

Example 1

[I] Expression for type I polypeptide and type II polypeptide, separation between type I polypeptide and type II polypeptide contained in thrombomodulin composition obtained by the expression, and recovery of each polypeptide

The polypeptide expression vector pSV2TMD1 coding for an amino acid sequence from the 1st position to the for an amino acid sequence from the 1st position to the 516th position shown in Fig. 1, was constructed. The construction method will be described below.

(1) Construction of plasmid pSV2TMJ2

Plasmid pSV2-dhfr (ATCC 37146) was completely digested with restriction enzymes HindIII and BglII to obtain a vector having early transcription promoter SV4 and transcription terminator SV40. Plasmid pUC18TMJ2 comprising a nucleotide sequence coding for 575 amino acids shown in Fig. 1, which plasmid was prepared in Referential Example 3-(13-2) of the Specification of the above-mentioned International Application Publication No. W088/05033, was partially digested with restriction enzyme HindIII and then, completely digested with restriction enzyme BamHI to obtain a DNA fragment of about 2900 bp. The DNA fragment was designated TMJ2. Subsequently, the DNA fragment of about 2900 bp and the above-obtained vector were ligated to each other by means of T4 DNA ligase to obtain plasmid pSV2TMJ2. The procedure for the construction of the plasmid pSV2TMJ2 is illustrated in Fig. 2.

The thus obtained plasmid pSV2TMJ2 is on deposit at the American Type Culture Collection (ATCC) under the Budapest Treaty and has been given accession No. 67283.

(2) Construction of plasmid pSV2TMD1

(a) Preparation of DNA fragment TMD1

The plasmid pSV2TMJ2 obtained in the above step (1) was completely digested with restriction enzyme NcoI to cleave the plasmid. Both ends of the cleaved plasmid were treated with E.coli DNA polymerase to make them blunt. Then, the cleaved plasmid was completely digested with restriction enzyme HindIII to obtain a DNA fragment of about 1900 bp. The thus obtained DNA fragment was designated TMJ3. On the other hand, phage M13mp19 (manufactured by Takara Shuzo Co., Ltd., Japan, catalog No. 3119) was digested with restriction enzymes HindIII and HincII to obtain a vector. The DNA fragment TMD3 was inserted in this vector to obtain recombinant plasmid M13mp19TMJ3.

Separately, a DNA probe for deletion (hereinafter referred to as "deleter") having the following nucleotide sequence was organo-chemically synthesized:

5'-GGAGGCCGCTCAGCCCGAATGCACG-3' (25mer).

The synthesized deleter was designated TMD.

Using the thus obtained deleter, TMD part of the above-obtained recombinant plasmid M13mp19TMJ13, which had a length of 177 nucleotides, was deleted by the technique of site-directed mutagenesis in accordance with the method described in Method in Enzymology, Vol. 100, 468(1983), Academic Press. Illustratively stated, 25 pmol of the deleter TMD and 10 pmol of M13 primer M3 (a universal primer manufactured by Takara Shuzo Co., Ltd., Japan, catalog No. 3831) were phosphorylated at their 5'-ends by means of T4 kinase. To the phosphorylated deleter and universal primer was added 0.5 pmol of a single-stranded DNA of the recombinant plasmid M13mp19TMJ3. The resultant mixture was heated at 95 °C for 5 minutes and then cooled to room temperature. Then, 5 units of E.coli DNA polymerase I (Klenow Fragment) and 10 units of T4 DNA ligase were added to the mixture and the mixture was incubated at 37 °C for 30 min to form a recombinant plasmid in the mixture. The thus obtained mixture was added to a culture of E.coli JM105 (manufactured by Pharmacia LKB, Sweden, catalog No. 27-1550) to transfect the E.coli with the recombinant plasmid. The E.coli was cultured on an agar plate at 37 °C overnight to thereby form plaques. The plaques were transplanted on a nitrocellulose filter and heated at 80 °C for 2 hours. Then, the nitrocellulose filter was subjected to prehybridization at 55 °C for 2 hours in a solution containing 6 x SET [0.9M NaCl, 180mM Tris buffer (pH8.0) and 6mM EDTA], 5 x Den harts' [0.1 % (w/v) Ficoll, 0.1 %·(w/v) polyvinyl pyrrolidone and 0.1 % (w/v) bovine serum albumin (BSA)], 0.1 % SDS and 100 µg/ml denatured DNA of a salmon sperm. Subsequently, the resultant nitrocellulose filter was subjected to hybridization at 55 °C for 2 hours in the solution as mentioned above in which a $^{32}P$-labeled TMD was used instead of the denatured DNA of a salmon sperm. Then, the nitrocellulose filter was washed with 6 x SSC solution (an aqueous solution containing 0.9M NaCl and 0.09M trisodium citrate) at room temperature twice each for 5 min. Further, the filter was washed with the same solution at successively elevated temperatures of 55 °C, 65 °C, and 75 °C. The above washing was conducted twice each for 5 min at the respective temperatures. Then, X-ray film XAR-5 (manufactured by Eastman Kodak Company, U.S.A.) was contacted with the resultant nitrocellulose filter at -80 °C overnight to expose the X-ray film. As a result, it was found that several tens of strongly exposed black spots were observed on the X-ray film. Each of the spots corresponded to the individual clone which had been transfected with the recombinant plasmid. Six clones were selected from the obtained clones, and the recombinant plasmid was isolated from each of the selected clones and analyzed with respect to its restriction sites and nucleotide sequence. The recombinant plasmids of the clones were found to be identical to each other with respect to the restriction sites and nucleotide sequence. The thus obtained recombinant plasmid was designated M13-TMD1. Further, the recombinant plasmid M13-TMD1 was found to contain a DNA fragment having a nucleotide sequence coding for the 18 amino acids of from the 1st amino acid to the 18th amino acid in Fig. 1 including an initiation coden (ATG) and, downstream thereof, a nucleotide sequence coding for a polypeptide comprised of 498 amino acids of from the 19th amino acid to the 516th amino acid in Fig. 1. The DNA fragment contained in the recombinant plasmid M13-TMD1 was designated TMD1.

(b) Construction of plasmid pSV2TMD1

The recombinant plasmid M13-TMD1 obtained in the above step (a) was completely digested with restriction enzymes HindIII and BamHI to isolate the DNA fragment TMD1 of about 1900 bp. On the other hand, the plasmid pSV2-dhfr (ATCC 37146) was completely digested with restriction enzymes HindIII and BglII to obtain a vector. This vector and the DNA fragment TMD1 were ligated with each other by means of T4 DNA ligase to obtain plasmid pSV2TMD1.

The thus obtained plasmid pSV2TMD1 and the plasmid pSV2-dhfr (ATCC No. 37146) were cotransfected into cell line CHO-dhfr$^-$ by means of the calcium phosphate method. The cell line CHO-dhfr$^-$ was obtained from Dr. L. Chasin and Dr. G.U. Chasin of Columbia University, U.S.A. The cells were cultured in Dulbecco's minimum essential medium (DMEM, manufactured by Flow Inc., U.S.A.) supplemented with proline and dialyzed fetal bovine serum (FCS., manufactured by GIBCO Inc., U.S.A.) so that the final concentrations thereof become 150 µg/µl and 10 %, respectively. Colonies appeared about a month later. Methotrexate (MTX, manufactured by WAKO PURE CHEMICAL Industries Ltd., Japan) was successively added to each of the cultured cells so that the resulting concentration of Methotrexate in each cell became 20 nM, 200 nM, 2 µM and 20 µM, thereby establishing cells for producing a polypeptide having the activity of promoting the thrombin-catalyzed activation of protein C. The cells were cultured in the above-mentioned medium at 37 °C for one week. The resultant cells were further cultured at 37 °C in substantially the same medium as mentioned above, with the exception of a decrease in concentration of fetal bovine serum to 1 %, to thereby obtain 10 liters of a culture.

Subsequently, 10 liters of the culture were applied to a Q Sepharose packed column (manufactured by Pharmacia LKB, Sweden) which had been equilibrated with 20 mM Tris-HCl buffer (pH7.4) containing 0.2 M sodium chloride to thereby adsorb an active fraction of the culture onto the Q Sepharose. After washing the column with the equilibrated buffer, the culture adsorbed on the Q Sepharose was eluted with a linear concentration gradient of NaCl (from 0.22 M to 1 M) to obtain two activity peaks as indicated in Fig. 3. The assay for the activity of promoting the thrombin-catalyzed activation of protein C was conducted according to the method as described later in [II]-(5). The unit of activity (1U) was defined as the activity of thrombomodulin producing 1 nmol of activated protein C per minute. Then, each of the two fractions [0.22 to 0.35 M in ionic strength (fraction Nos. 8 to 18 in Fig. 3) and 0.55 M to 0.75 M in ionic strength (fraction Nos. 38 to 58 in Fig. 3)] was pooled and subjected to dialysis for desalting.

Thereafter, each of the two fractions was separately applied to a diisopropylphosphate-thrombin-Sepharose packed column equilibrated with 20 mM phosphate buffer (pH7.4) containing 0.2 M NaCl to adsorb the active portion thereto. After washing the column with the same buffer as used for the equilibration, the active portion was eluted with phosphate buffer (pH7.4) containing 1.0 M NaCl to obtain an active fraction. Further, the resultant active fraction was concentrated. The concentrate was applied to a column packed with Sephacryl S-300 (manufactured by Pharmacia LKB, Sweden) equilibrated with phosphate-buffered saline (PBS), and developed with the same phosphate buffer to obtain active fractions.

Each of the active fractions was pooled and concentrated.

The purified concentrate obtained from the fraction eluted from the Q Sepharose packed column at lower salt concentrations (0.22 - 0.35 M NaCl) was type I polypeptide, and the purified concentrate obtained from the fraction eluted at higher salt concentrations (0.55-0.75 M NaCl) was Type II polypeptide. Final concentrations of the resultant type I polypeptide solution and type II polypeptide solution were 1.74 mg/ml and 1.59 mg/ml, respectively.

[II] Characterization

The type I polypeptide and type II polypeptide obtained in the above procedure [II] were examined with respect to the following properties.

(1) Molecular weight

The molecular weight of each of type I polypeptide and type II polypeptide was determined by electrophoresis. The type I polypeptide and type II polypeptide were applied to sodium dodecyl sulfate (SDS)-7.5 % polyacrylamide electrophoresis (PAGE) under reduced conditions (Fig. 4). The type I polypeptide was detected as a single band of about 100 kilodaltons, while the type II polypeptide was very heterogeneous and detected as a smear band of about 100 kilodaltons or more. Moreover, the fractions were substantially not contaminated with each other.

(2) Amino acid sequences

The N-terminal amino acid residue of each of the type I polypeptide and type II polypeptide was analyzed using an amino acid sequence analyzer Model 470A manufactured by Applied Biosystems, Inc., U.S.A. From the N-terminal, Edman degradation was successively performed to isolate phenylthiohydantoin amino acids. The thus isolated amino acids were analyzed using high performance liquid chromatography apparatus SP8100 manufactured by Spectrophysics Inc., U.S.A., and ZORVAX ODS packed column manufactured by E. I. Du Pont De NEMOURS AND COMPANY, U.S.A. to determine the amino acid sequence.

The results showed that the N-terminal amino acid sequences of both of the polypeptides completely coincided with each other as shown in Fig. 5. Each of the polypeptides was provided with two different N-termini attributed to the processing of each signal polypeptide portion at two sites thereof (a site between the 16th amino acid and the 17th amino acid, and another site between the 18th amino acid and the 19th amino acid in Fig. 1).

(3) Isoelectric point

The measurement of isoelectric point was carried out with an isoelectric focusing apparatus (Multiphor II, manufactured by Pharmacia LKB, Sweden).

Each of the type I polypeptide and type II polypeptide dissolved in deionized water and marker proteins, were loaded on a gel and allowed to migrate at maximum 10 mA (Max. 700V) for 3 hours while keeping the temperature at 5 °C. After the migration, the resultant gel was stained with Coomassie Brilliant Blue. From the mobilities of the marker proteins indicated below, each pH gradient was determined, and from the mobilities of two types of polypeptides, the isoelectric point (pI) of each polypeptide was determined (Fig. 6).

| | |
|---|---|
| Patent blue Sodium Salt | pI = 2.4 |
| Pepsinogen | pI = 2.8 |
| Amyloglucosidase | pI = 3.5 |
| Methyl Red | pI = 3.75 |
| Acetylated Cytochrome C | pI = 3.95 |

Further, unstained gel was cut into slices each having a predetermined thickness, and subjected to protein elution. The activity of promoting the thrombin-catalyzed activation of Protein C was assayed to obtain the results shown in Fig. 6. That is, the activity of the type I polypeptide was detected in a pH range of 3.4 ± 0.2 and the activity of the type II polypeptide was detected in a pH range of not greater than 3.2. In Fig. 6, the straight line indicates the pH gradient, and the bar indicates the activity of each fraction eluted from the sliced gel.

From the above results, the isoelectric point of the type II polypeptide was estimated at pH 3.2 or lower, and that of the type I polypeptide at pH 3.4 ± 0.2.

(4) Binding property to thrombin

The affinity of each of the type I polypeptide and type II polypeptide to thrombin was measured using diisopropyl phosphate-thrombin-Sepharose [J. Biol. Chem. Vol. 245, pp. 3059-3065 (1970)].

Each of the type I polypeptide and type II polypeptide was applied to a diisopropyl phosphate-thrombin-Sepharose packed column equilibrated with a 20 mM phosphate buffer containing 0.2 M NaCl. Almost all active fractions were adsorbed onto the column, and completely eluted with a phosphate buffer containing 1 M NaCl.

(5) Activity of promoting the activation of Protein C

The activity of promoting the thrombin-catalyzed activation of protein C of each of the type I polypeptide and type II polypeptide was assayed by the following method.

3 µl of thrombin (manufactured by SIGMA Chemical Co., U.S.A., human-derived, 20 ng/µl), 5 µl of type I polypeptide or type II polypeptide solution, 5 µl of 10 x assay buffer [0.5 M Tris-HCl buffer (pH 8.5) containing 1 M NaCl, 30 mM $CaCl_2$ and 1 % bovine serum albumin] and 29.5 µl of distilled water were mixed. The mixture was allowed to stand at 37 °C for 5 min, and 75 µl of protein C (bovine-derived, 0.2 µg/µl) was added to the mixture to react at 37 °C for 30 min. The reaction was stopped by adding 6.25 µl of a stop solution [20 mM Tris-HCl buffer (pH 7.5) containing 100 mM NaCl, $0.3A_{230}$ anti-thrombin III and 100 µg/ml heparin].

The activity of promoting the activation of protein C was assayed by the following method. To the above-obtained reaction mixture was added 10 µl of Boc-Leu-Ser-Thr-Arg-MCA (MCA: 4-methyl-coumarinyl-7-amide, manufactured by Peptide Institute, Inc., Japan, 3112-V, 5 mg/ml), 5 µl of 5 M cesium chloride, 495 µl of substrate reaction buffer (50 mM Tris-HCl buffer containing 100 mM NaCl) to react at 37 °C for 20 min. The reaction was stopped by adding 55 µl of acetic acid. Then, the concentration of liberated fluorescent agent 7-amino-4-methyl-coumarin (AMC) was measured by a fluorescent spectrophotometer at an excited wavelength of 380 nm and at a luminous wavelength of 440 nm. The activity of promoting the activation of protein C was determined therefrom, using a separately prepared calibration curve indicating the relationship between the concentration of AMC and the amount of activated protein C produced.

The results confirmed that both of the type I polypeptide and the type II polypeptide exhibited high activity.

[III] Analysis of the sugar chain structure of type II polypeptide

The type II polypeptide obtained in the above procedure [I] was analyzed with respect to a sugar chain structure.

(1) Identification of sugar chain

The identification of the sugar chain attached to the type II polypeptide was conducted by digesting the polypeptide with various degradation enzymes for sugar chain. When the type II polypeptide was treated with chondroitinase ABC (a degradation enzyme for chondroitin, chondroitin sulfate, dermatan sulfate, and hyaluromic acid) (manufactured by SEIKAGAKU KOGYO Co., Ltd., Japan), the molecular weight of the treated type II polypeptide became lower on the SDS-PAGE, which was identical with the molecular weight of type I polypeptide (Fig. 7, No. 3 lane). Therefore, it is apparent that the type II polypeptide is comprised of the type I polypeptide having, attached thereto, at least one enzyme for degrading chondroitin, chondroitin sulfate, dermatan sulfate and hyaluronic acid.

When the type II polypeptide was treated with heparinase (an enzyme for degrading only heparin, manufactured by SEIKAGAKU KOGYO Co., Ltd., Japan), heparitise (an enzyme for degrading only heparan sulfate, manufactured by SEIKAGAKU KOGYO Co., Ltd., Japan) or hyaluronidase (an enzyme for degrading only hyaluronic acid, manufactured by SEIKAGAKU KOGYO Co., Ltd., Japan), any change in molecular weight of the treated polypeptide was not observed (Fig. 7, lane Nos. 4, 5, and 6). Hence, the substance attached to the type II polypeptide is neither heparin, nor heparan sulfate, nor hyaluronic acid, but one selected from chondroitin, chondroitin sulfate and dermatan sulfate.

Further, the type II polypeptide was treated with chondroitinase AC (a degradation enzyme for chondroitin, chondroitin sulfate and hyaluronic acid, manufactured by SEIKAGAKU KOGYO Co., Ltd., Japan). The molecular weight of the treated polypeptide was identical with that of the type I polypeptide (Fig. 8), as in the treatment of the type II polypeptide with chondroitinase ABC. Since chondroitinase AC and chondroitinase ACII do not degrade dermatan sulfate, it is apparent that the sugar chain attached to the polypeptide of the type II polypeptide is chondroitin and/or chondroitin sulfate.

Still further, the sugars constituting chondroitin or chondroitin sulfate attached to the type II polypeptide were identified by thin-layer chromatography.

A degradation product of 50 µg of the type II polypeptide with chondroitinase ABC and various standard reference materials, such as unsaturated chondrodisaccharides ΔDi-4S (Δ4,5-glucuronide-N-acetylgalactosamine-4 sulfate), ΔDi-6S (Δ4,6-glucuronide-N-acetylgalactosamine-6 sulfate), and ΔDi-0S (Δ4,5-glucuronide-N-acetylgalactosamine) (each manufactured by SEIKAGAKU KOGYO CO., Ltd., Japan), were spotted on a cellulose plate (manufactured by Merck Co., Germany), and developed overnight with a desalting solvent, n-butanol-ethanol-water (52:32:16, v/v). The plate was dried, and the spots were developed with n-butanol-acetic acid-1N ammonia (2:3:1, v/v) as a developing solvent for 7 hours. Unsaturated sugar was detected by the ultraviolet absorption method. The results showed that a substance having the same mobility as ΔDi-4S was contained in the chondroitinase-treated type II polypeptide as shown in Fig. 9. Therefore, the main component of the chondroitin sulfate attached to the type II polypeptide was proved to be chondroitin-4-sulfate.

(2) Measurement of the content of chondroitin and/or chondroitin sulfate

The content of chondroitin or chondroitin sulfate attached to the type II polypeptide was determined in terms of a uronic acid content by measuring the content of uronic acid contained in both of chondroitin and chondroitin sulfate.

The quantitative analysis of uronic acid was carried out by the following method, using glucuronic acid (manufactured by Aldrich Chemical Co., Inc., U.S.A.) as a standard reference material. That is, 0.2 ml of type II polypeptide was gently layered onto 1 ml of sodium borate solution (0.95 g of sodium borate/100 ml of sulfuric acid) cooled with iced water. The layered mixture was shaken first slowly and then violently, while cooling enough to keep the temperature of the mixture at a level not higher than room temperature. The mixture was heated in a boiling water bath for 10 min, followed by cooling to room temperature in running water. A carbazole solution (12.5 mg of carbazole/10 ml of ethanol) was added to the mixture and shaken. The mixture was then heated in a boiling water bath for 15 min and cooled to room temperature. The absorbance of the resultant mixture was measured at 530 nm.

The uronic acid content of the type II polypeptide obtained in the above procedure [I] was 100 µg per mg of amino acid portion of the polypeptide. [IV] Comparison of physiological properties between type I polypeptide and type II polypeptide

The type I polypeptide and the type II polypeptide obtained in the above procedure [I] were compared to each other with respect to various physiological properties.

Thrombomodulin has an anticoagulant activity, thereby prolonging various blood clotting times. In the present example, the type I polypeptide and the type II polypeptide obtained in the above procedure [I] and the chondroitinase-treated type II polypeptide were compared to one another with respect to the effect of prolonging the thrombin clotting time related to common factors in the blood coagulation cascade and the effect of prolonging the activated partial thromboplastin time related to intrinsic factors in the blood coagulation cascade.

(1) Measurement of Thrombin Clotting Time (TCT)

Thrombin clotting time (TCT) was measured using Coagulometer KC10A manufactured by Heinrich Amelung GmbH of Germany. Human control plasma Ci-trol I (manufactured by International Reagents Corporation, Japan) was diluted 10-fold with veronal buffer (Owren, pH 7.35) (manufactured by International Reagents Corporation, Japan), and kept at 37 °C. 25 µl of human thrombin (40 U/ml) (manufactured by SIGMA Chemical Co., USA) was mixed with 25 µl of individual type II polypeptides of different concentrations which were prepared by diluting the type II polypeptide obtained in the above procedure [I] with physiological saline to concentrations of 4, 6, 8, 10, 20, 40, 60 and 80 µg/ml. The mixture was then incubated at 37 °C for 2 min. 200 µl of the above diluted human control plasma was put in a sample cup containing a steel ball, to which 50 µl of the above mixed solution was added. The time from the moment that the plasma began to coagulate to the moment that the steel ball began to move, was measured. The same test was also conducted with respect to the type I polypeptide obtained in the above procedure [I] and the chondroitinase-treated type II polypeptide. The quantitative analysis of protein was conducted by the Lowry-Folin method. The results are shown in Fig. 10.

When only thrombin was added to the plasma, the plasma was coagulated at once. Both of the type II polypeptide and type I polypeptide prolonged the thrombin clotting time, depending on the concentration. It was noted that for prolonging the thrombin clotting time for a given time the type I polypeptide was required to be used in a large amount as compared to the amount of the type II polypeptide. The chondroitinase-treated type II polypeptide showed the same results as obtained with respect to the type I polypeptide.

Exactly the same results were obtained when the test was carried out in substantially the same manner as above except that fibrinogen (manufactured by SIGMA Chemical Co., USA) was used instead of the human control plasma (Fig. 11).

(2) Measurement of activated partial thromboplastin time (APTT)

Activated partial thromboplastin time (APTT) was measured using Coagulometer KC 10A manufactured by Heinrich Amelung GmbH, Germany. Human control plasma Ci-trol I (manufactured by International Reagents Corporation, Japan) was dissolved in 1 ml of deionized water, and the resultant solution was kept at 37 °C. 100 µl of the plasma was put in a sample cup containing a steel ball, to which 20 µl of type II polypeptide obtained in the above procedure [I] (diluted with physiological saline to the respective concentration of 3, 7, 10, 20, 40, 50, 55, 60, 65 and 70 µg/ml) was added. The mixture was incubated at 37 °C for 2 min, and 100 µl of actin (manufactured by International Reagents Corporation, Japan) as a phospholipid was added thereto and further incubated at 37 °C for 3 min. Coagulation was initiated by adding 100 µl of 20 mM calcium chloride solution (manufactured by International Reagents Corporation, Japan). The time from the initiation to the completion of the coagulation of the plasma was measured by detecting the movement of the steel ball. The same test was carried out with respect to the type I polypeptide obtained in the above procedure [I] and the chondroitinase-treated type II polypeptide. The results are shown in Fig. 12.

The plasma, to which the type II polypeptide was not added, was coagulated at once. However, the addition of the type II polypeptide to the plasma prolonged the activated partial thromboplastin time, depending on the concentration. The type I polypeptide also prolonged the activated partial thromboplastin time, depending on the concentration, but the amount thereof required to prolong the activated partial thromboplastin time for a given time was larger than that of the type II polypeptide. The chondroitinase-treated type II polypeptide exhibited substantially the same results as the type I polypeptide.

(3) Measurement of the affinity with thrombin

Thrombomodulin binds to thrombin to exhibit an anti-coagulation activity. The type II polypeptide, the type I polypeptide and the chondroitinase-treated type II polypeptide were compared to one another with respect to the affinity with thrombin by measuring the respective dissociation constants (Kd) of the dissociation of thrombin-polypeptide.

3 µl of 0.4 ng/µl human thrombin (manufactured by SIGMA Chemical Co., USA) dissolved in buffer [20 mM Tris-HCl (pH 7.5) containing 0.1 M NaCl, 0.1 % Lubrol PX, 0.1 % sodium azide, and 0.001 % BSA (bovine serum albumin)], 5 µl of the individual type II polypeptide which was obtained in the above procedure [I] and diluted with the above buffer to the respective concentration (0.2, 0.4, 1.0, 2.5 and 10 µg/ml), 5 µl of reaction buffer [0.5 mM Tris-HCl (pH 8.5) containing 0.1 M NaCl, 30 mM $CaCl_2$, and 0.1 % BSA] and 29.5 µl of deionized water, were mixed together and incubated at 37 °C for 5 min. 7.5 µl of protein C (0.2 µg/µl) prepared from bovine blood was added to the resultant mixture and further incubated at 37 °C for 30 min. After the incubation, 6.25 µl of reaction stop solution [20 mM Tris-HCl (pH 7.5) containing 0.1 M NaCl, 300 µg/ml bovine AT III (anti-thrombin III, prepared from bovine blood), and 100 µl/ml heparin (manufactured by WAKO PURE CHEMICAL Industries Ltd., Japan)] was added and further incubated at 37 °C for 5 min to stop the reaction.

The following reaction was carried out in order to determine the amount of activated protein C produced. 510 µl of buffer [50 mM Tris-HCl (pH 8.5) containing 0.1 M NaCl, 50 mM CsCl, and 0.1 mg/ml BoC-Leu-Ser-Thr-Arg-MCA (manufactured by Peptide Institute, Inc., Japan, 3112-V, 5 mg/ml)] containing a substrate was added to the above reaction mixture and allowed to react at 37 °C for 20 min. 55 µl of acetic acid was added to the reaction mixture to stop the reaction. The amount of liberated fluorescence agent AMC was determined by exciting the AMC at a wavelength of 380 nm and measuring the fluorescence intensity at a wavelength of 440 nm. The amount of the activated protein C produced per ml of the reaction mixture per minute was determined using a separately prepared calibration curve.

The dissociation constant (Kd) of the dissociation of thrombin-polypeptide was determined by plotting the reciprocal of the amount of activated protein C produced versus the reciprocal of the concentration of the tested material. The same test was carried out with respect to the type I polypeptide obtained in the above procedure [I] and the chondroitinase-treated type II polypeptide. The Kd value was determined from a point of intersection of each of the straight lines obtained by the plotting with the X-axis (Fig. 13).

As a result, it was found that the Kd value of the type II polypeptide was 2.9 x $10^{-9}$M, that of the type I polypeptide was 8.9 x $10^{-9}$M, and that of the chondroitinase-treated type II polypeptide was 1.6 x $10^{-8}$M. That is, the type II polypeptide was considered to have a high affinity with thrombin as compared to the type I polypeptide.

(4) Measurement of platelet aggregation inhibiting activity

Thrombomodulin inhibits thrombin-catalyzed aggregation of platelets. The three types of polypeptides were compared to one another with respect to the platelet aggregation inhibiting activity.

The platelet aggregation inhibiting activity was measured with a platelet aggregation measuring device (HEMA TRACER IV sold by NIKO Bioscience, Inc., Japan). 40 µl of a mixture of human thrombin (manufactured by SIGMA Chemical Co., USA, 12.5 U/ml, 20 µl) previously incubated at 37 °C for 2 min and 20 µl of the individual of the type II polypeptides of different concentrations (6.4, 8.0, 10.6 and 15.9 µg/ml) obtained in the above procedure [I] was added to 180 µl of plasma containing platelets (3 x $10^5$ platelets/µl) prepared from normal human blood. Platelet aggregation was observed by tracing a decrease in turbidity with time, using a platelet deficiency plasma as a control. The same test was also carried out with respect to the type I polypeptide obtained in the above procedure [I] and the chondroitinase-treated type II polypeptide.

The results are shown in Fig. 14. When the platelets were stimulated with only thrombin, the platelets aggregated at once. However, in the presence of 1.4 µg/ml of type II polypeptide, the aggregation of the platelets was completely inhibited. The platelet aggregation inhibition was dependent on the concentration of the type II polypeptide. With respect to the type I polypeptide as well, the concentration dependency was observed. However, with the type I polypeptide, complete inhibition of the platelet aggregation was not attained irrespective of the presence thereof in the same amount as the amount of the type II polypeptide which was used for completely inhibiting the platelet aggregation. The chondroitinase treated type II polypeptide exhibited the same results as those of the type I polypeptide.

(5) Measurement of the inhibition of the thrombin-catalyzed formation of factor Va

Thrombin activates factor V to form factor Va. Thrombomodulin inhibits the formation of the factor Va. The three types of polypeptides were compared to one another with respect to the activity of inhibiting the formation of factor Va.

5 µl of human thrombin (25 ng/µl) (manufactured by SIGMA Chemical Co., USA) and 5 µl of the individual of type II polypeptides of different concentrations (25, 50, 100, 200, 400 and 800 µg/ml) obtained in the above procedure [I], were mixed together and incubated at 37 °C for 5 min. Thereafter,10 µl of factor V (0.38 µg/ml) (manufactured by Enzyme Research Laboratories, Inc., USA) was added to the mixture and further incubated at 37 °C for 20 min. On the other hand, 5 µl of factor Xa (0.25 U/ml) (manufactured by SIGMA Chemical Co., USA) and 5 µl of phospholipid (2 mg/ml) (manufactured by SIGMA Chemical Co., USA, plain extract type III) were mixed with 50 µl of reaction buffer [20 mM Tris-HCl (pH 7.5) containing 150 mM NaCl, 0.1 % BSA and 5 mM NaCl]. 5 µl of prothrombin (10 U/ml) (manufactured by SIGMA Chemical Co., USA) was added to the mixture, and preheating was initiated. The mixture was incubated at 37 °C for 5 min, and 10 µl of the above-prepared thrombin-polypeptide-factor V mixed solution was added thereto and further incubated for 5 min.

Then, 10 µl of 0.15 M EDTA was added thereto to stop the reaction, and further 1 ml of Boc-Val-Pro-Arg-MCA as a substrate of thrombin [manufactured by Peptide Institute, Inc., 100-fold diluted solution of 1 bial (1 ml) with 50 mM Tris-HCl (pH 8.5) containing 100 mM NaCl] was added and incubated at 37 °C for 5 min. After the incubation, 110 µl of acetic acid was added to stop the reaction. The amount of fluorescence agent AMC liberated in the reaction mixture was determined by exciting at a wavelength of 380 nm and measuring a fluorescence intensity at a wavelength of 440 nm. The formation ratio of factor Va was calculated by the use of a previously prepared calibration curve showing the relationship between the amount of factor Va and the thrombin activity. The same test was carried out with respect to the type I polypeptide obtained in the above procedure [I] and the chondroitinase treated type II polypeptide.

The results, as shown in Fig. 15, indicate that the factor Va formation-inhibiting activity of the type II polypeptide was higher than that of the type I polypeptide. The chondroitinase treated type II polypeptide had substantially the same activity as that of the type I polypeptide.

Example 2

Substantially the same procedure as in Example 1-[1] was repeated except that cells of CHO-KI line-(ATCC, CCLD 61) were employed instead of the cells of CHO-dhfr⁻ line, to thereby obtain type I polypeptide and type II polypeptide. The properties of the polypeptides were examined in substantially the same manner as in Example 1-[II], [III] and [IV] to obtain substantially the same results as obtained in Example 1-[II], [III] and [IV].

Example 3

[I] Expression for type I polypeptide and type II polypeptide comprising minimum activity expression sites, separation between type I polypeptide and type II polypeptide contained in the thrombomodulin composition obtained by the expression, identification of the sugar chain, and measurement of the activities

(1) Construction of plasmid pSV2TMD6

DNA fragment designated as TMD6 was obtained in the same manner as in Example 1-[I] except that use was made of the recombinant plasmid M13-TMD1 described in Example 1-[I] and a deleter $TMd_8$ having the following nucleotide sequence

5′-GAAGCACGGGTCGGGGAACCCCAGG-3′ (25 mer)

as a DNA probe for deletion, to thereby perform the deletion of 1044 bp. The DNA fragment TMD6 had a nucleotide sequence including from the initiation codon (ATG) a nucleotide sequence coding for a polypeptide comprised of amino acid residues up to the 18th position and amino acid residues from the 367th position to the 516th position in Fig. 1. The DNA fragment TMD6 was completely digested with restriction enzymes HindIII and BamHI to isolate a DNA fragment of about 700 bp.

On the other hand, the plasmid pSV2-dhfr was completely digested with restriction enzymes HindIII and BglII to obtain a vector. This vector and the above DNA fragment of about 700 bp were ligated with each other by means of T4 DNA ligase to obtain plasmid pSV2TMD6.

(2) Cotransfection of expression vector pSV2TMD6 and plasmid pSV2-dhfr into cell $CHO\text{-}dhfr^-$.

Plasmid pSV2TMD6 and plasmid pSV2-dhfr were cotransfected into cell $CHO\text{-}dhfr^-$ by the calcium phosphate method. Cultivation was conducted using, as a medium, DMEM to which proline and dialyzed FCS had been added so that final concentration thereof were 150 µg/ml and 10 %, respectively. About a month later, colonies appeared, thereby obtaining cells capable of producing thrombomodulin polypeptide.

(3) Comparison between two types of polypeptides produced by the expression vector pSV2TMD6.

From 10 liters of the culture of the above cells, type I polypeptide and type II polypeptide were separated from each other and purified according to substantially the same procedure as in Example 1-[I].

As a result, type II polypeptide having chondroitin and/or chondroitin sulfate attached thereto and type I polypeptide having none of them attached thereto were obtained as in Example 1-[I].

The purified polypeptides were compared to each other with respect to thrombin clotting time and activated partial thromboplastin time in the same manner as in Example 1-[IV]-(1) and -(2). As shown in Fig. 16 and Fig. 17, substantially the same results was obtained as in Example 1-[IV]. That is, with respect to the thrombin clotting time, although the addition of only thrombin to the plasma caused prompt coagulation thereof, both of the type II polypeptide and the type I polypeptide prolonged the thrombin clotting time, depending on the concentration. However, the amount required to prolong the thrombin clotting time for a given time was larger in the case of the type I polypeptide than in the case of the type II polypeptide. With respect to the chondroitinase treated type II polypeptide, substantially the same results as those obtained with respect to the type I polypeptide were obtained.

With respect to the activated partial thromboplastin time, when the type II polypeptide was not added, the plasma coagulated at once. However, by adding the type II polypeptide, the activated partial thromboplastin time was prolonged, depending on the concentration. The type I polypeptide also prolonged the activated partial thromboplastin time, depending on the concentration, however, for prolonging the APTT for a given time, it is required to use larger amount than the amount of the type II polypeptide. The chondroitinase treated type II polypeptide exhibited substantially the same results as those obtained with respect to the type I polypeptide.

Example 4

Substantially the same procedure as in Example 3 was repeated except that cells of CHO-K1 line were used instead of cells of CHO-dhfr$^-$ line, to thereby obtain type I polypeptide and type II polypeptide. The properties thereof were examined in the same manner as in Example 3 to obtain substantially the same results as in Example 3.

Example 5

A variant (Ala type homologous variant) which had an amino acid sequence of 498 amino acids of from the 19th amino acid to the 516th amino acid in Fig. 1, in which only the 473rd amino acid, Val, was replaced by Ala, was compared with the Val prototype with respect to physiological activities.

(1) Construction and expression of plasmid pSV2TMD11

The plasmid pSV2TMD1 obtained by substantially the same procedure as in Example [I] was digested with restriction enzyme XmaIII, and was subjected to low melting point agarose gel electrophoresis to separate a DNA fragment of 0.57 kbp containing Val at the 473rd position from a remaining DNA fragment of 5.38 kbp. Thus, the DNA fragment of 5.38 kbp was isolated. On the other hand, separately, plasmid pUC 13-1 containing a thrombomodulin gene having Ala at the 473rd position which was obtained by cloning from human lymphocyte, was cleaved with restriction enzyme XmaIII to isolate a DNA fragment of 0.57 kbp containing Ala at the 473rd position by low melting point agarose gel electrophoresis. The thus isolated DNA fragment of 5.38 kbp and the DNA fragment of 0.57 kbp were ligated with each other by means of T4 DNA ligase to obtain plasmid pSV2TMD11.

Plasmids pSV2TMD1 and pSV2TMD11 were individually transfected into cells of COS-1 line (ATCC, CRL 1650) by the electroporation method. The transfected cells were cultured in DMEM medium containing 10 % FCS on a plastic petri dish having a diameter of 10 cm for 24 hours. Thereafter, the medium was removed. The culture was washed with PBS buffer and further cultured in DMEM medium containing no serum for 48 hours, followed by recovery of the supernatant.

(2) Comparison of physiological activities

The type II polypeptide and the Ala type homologous variant contained in the recovered supernatant were separately purified using the diisopropylphosphate-thrombin-Sepharose column. The concentration of thrombomodulin in each of the purified samples was determined by the sandwich ELISA method using a monoclonal antibody. The amounts of the obtained type II polypeptide and Ala type homologous variant were 152 µg and 222 µg, respectively. The thus obtained samples were compared with respect to the dissociation constant Kd of the dissociation of thrombin-polypeptide and the apparent Michaelis constant Km to protein C. The Km value was determined as follows. The amounts of the activated protein C produced at different concentrations of protein C (0, 1.0, 2.5, 5.0 and 10 µM) were measured substantially in accordance with the method for measuring the activity of promoting the activation of protein C as described in Example 1-[II]-(5). Then, the reciprocals thereof were plotted to determine the Km value. Further, the Kd value was determined as follows. Similarly, the amounts of activated protein C produced at different concentrations of thrombomodulin (0, 1.0, 2.5, 5.0 and 10 µM) were measured, and the reciprocals thereof were plotted to determine the Kd value.

The results are shown in Fig. 18 and Fig. 19, respectively. As apparent from the Figures, there was no significant difference in Km (= 3.2 µM) and Kd (= 1.7 NM) between the type II polypeptide and the Ala type homologous variant thereof.

Example 6

6 ml of a solution of the individual of type II polypeptides which were separately obtained in Example 1-[I] and in Example 3, 20 mg of purified gelatin and 100 mg of mannitol, were put in a sterile vial, prefrozen at -35 °C for 2 hours, subjected to primary drying under a vacuum of 0.075 Torr for 33 hours, and subjected to secondary drying under a vacuum of 0.03 Torr for 5 hours, to thereby produce 2 types of injection vials.

Brief Description of the Drawings

Fig. 1 shows the entire amino acid sequence of thrombomodulin;

Fig. 2 shows a flow chart illustrating the construction of plasmid pSV2TMJ2;

Fig. 3 shows an elution pattern obtained by applying the conditioned medium of CHO-dhfr$^-$ cells containing plasmid pSV2TMD1 onto Q-Sepharose, followed by elution using a linear concentration gradient of 0.22-1 M NaCl;

Fig. 4 is a schematic illustration of an SDS-polyacrylamide electrophoresis pattern of the type I polypeptide and the type II polypeptide;

Fig. 5 shows the N-terminal amino acid sequences of the type I polypeptide and the type II polypeptide;

Fig. 6 shows the results of an isoelectric focusing of the type I polypeptide and the type II polypeptide;

Fig. 7 is a schematic illustration of an SDS-polyacrylamide electrophoresis pattern of the type II polypeptide treated with various glycosidases;

Fig. 8 is a schematic illustration of an SDS-polyacrylamide electrophoresis pattern of the type II polypeptide treated with various glycosidases;

Fig. 9 is a schematic illustration of a cellulose thin-layer chromatography pattern of the type II polypeptide treated with chondroitinase ABC;

Fig. 10 shows thrombin clotting times (obtained using a control plasma) with respect to the type I polypeptide, the type II polypeptide and chondroitinase-treated type II polypeptide;

Fig. 11 shows thrombin clotting times (obtained using fibrinogen) with respect to the type I polypeptide, the type II polypeptide and chondroitinase-treated type II polypeptide;

Fig. 12 shows activated partial thromboplastin times (APTT) with respect to the type I polypeptide, the type II polypeptide and chondroitinase-treated type II polypeptide;

Fig. 13 shows a Lineweaver-Burk plot for determining the dissociation constant Kd values of the type I polypeptide, the type II polypeptide and chondroitinase-treated type II polypeptide;

Fig. 14 shows the platelet coagulation inhibiting activities of the type I polypeptide, the type II polypeptide and chondroitinase-treated type II polypeptide;

Fig. 15 shows factor Va-formation inhibiting activities of the type I polypeptide, the type II polypeptide and chondroitinase-treated type II polypeptide;

Fig. 16 shows the thrombin clotting times (TCT) with respect to the type I polypeptide, the type II polypeptide and chondroitinase-treated type II polypeptide which are produced by using plasmid pSV2TMD6;

Fig. 17 shows the activated partial thromboplastin times (APTT) with respect to the type I polypeptide, the type II polypeptide and chondroitinase-treated type II polypeptide which are produced by using plasmid pSV2TMD6;

Fig. 18 shows the relationships between the concentration of protein C and the amount of activated protein C produced, with respect to the type II polypeptide and Ala type homologous variant thereof; and

Fig. 19 shows the relationships between the concenprotein C produced, with respect to the type II polypeptide and Ala type homologous variant thereof.

Industrial Applicability

The human thrombomodulin polypeptide (type II thrombomodulin polypeptide) of the present invention which has a sugar chain comprising chondroitin and/or chondroitin sulfate attached to the peptide chain thereof, exhibits excellent anticoagulant, platelet aggregation inhibiting and thrombolytic activities. Hence, this type II thrombomodulin polypeptide can be used, for example, for the treatment and prevention of diseases, such as myocardial infraction, thrombosis, embolism, obstruction of peripheral blood vessels, arteriosclerosis obliterans, disseminated intravascular coagulation (DIC) syndrome, angina pectoris, transient ischemic attack, toxemia of pregnancy and the like. Especially when such diseases are in a state where it is required to exhibit higher physiological activities in a short period, for example, when the diseases are in an acute phase, a pharmaceutical composition containing, as an active ingredient, the type II thrombomodulin polypeptide of the present invention having high activities is extremely effective.

## Claims

1. An isolated physiologically active human thrombomodulin polypeptide which has the activity of promoting the thrombin-catalyzed activation of protein C and which has a sugar chain comprising chondroitin and/or chondroitin sulfate attached to the peptide chain of said polypeptide.

2. The human thrombomodulin polypeptide according to claim 1, wherein said peptide chain comprises an amino acid sequence represented by the formula (I):

```
Val Asp Pro Cys Phe Arg Ala Asn Cys
Glu Tyr Gln Cys Gln Pro Leu Asn Gln
Thr Ser Tyr Leu Cys Val Cys Ala Glu
Gly Phe Ala Pro Ile Pro His Glu Pro
His Arg Cys Gln Met Phe Cys Asn Gln
Thr Ala Cys Pro Ala Asp Cys Asp Pro
Asn Thr Gln Ala Ser Cys Glu Cys Pro
Glu Gly Tyr Ile Leu Asp Asp Gly Phe
Ile Cys Thr Asp Ile Asp Glu Cys Glu
Asn Gly Gly Phe Cys Ser Gly Val Cys
His Asn Leu Pro Gly Thr Phe Glu Cys
Ile Cys Gly Pro Asp Ser Ala Leu Val
Arg His Ile Gly Thr Asp Cys  ... (I)
```

or a homologous variant of said polypeptide, having the activity of promoting the thrombin-catalyzed activation of protein C.

3. The human thrombomodulin polypeptide according to claim 2, wherein said peptide chain further comprises an amino acid sequence represented by the formula (II):

```
Asp Ser Gly Lys Val Asp Gly Gly Asp Ser
Gly Ser Gly Glu Pro Pro Pro Ser Pro Thr
Pro Gly Ser Thr Leu Thr Pro Pro Ala Val
Gly Leu Val His Ser Gly ... (II).
```

attached to said amino acid sequence of formula (I) at its C-terminus, or a homologous variant of said polypeptide, having the activity of promoting the thrombin-catalyzed activation of protein C.

4. The human thrombomodulin polypeptide according to claim 2, wherein said peptide chain further comprises an amino acid sequence represented by the formula (III):

Ala Pro Ala Glu Pro Gln Pro Gly Gly Ser
Gln Cys Val Glu His Asp Cys Phe Ala Leu
Tyr Pro Gly Pro Ala Thr Phe Leu Asn Ala
Ser Gln Ile Cys Asp Gly Leu Arg Gly His
Leu Met Thr Val Arg Ser Ser Val Ala Ala

Asp Val Ile Ser Leu Leu Leu Asn Gly Asp
Gly Gly Val Gly Arg Arg Arg Leu Trp Ile
Gly Leu Gln Leu Pro Pro Gly Cys Gly Asp
Pro Lys Arg Leu Gly Pro Leu Arg Gly Phe
Gln Trp Val Thr Gly Asp Asn Asn Thr Ser
Tyr Ser Arg Trp Ala Arg Leu Asp Leu Asn
Gly Ala Pro Leu Cys Gly Pro Leu Cys Val
Ala Val Ser Ala Ala Glu Ala Thr Val Pro
Ser Glu Pro Ile Trp Glu Glu Gln Gln Cys
Glu Val Lys Ala Asp Gly Phe Leu Cys Glu
Phe His Phe Pro Ala Thr Cys Arg Pro Leu
Ala Val Glu Pro Gly Ala Ala Ala Ala Ala
Val Ser Ile Thr Tyr Gly Thr Pro Phe Ala
Ala Arg Gly Ala Asp Phe Gln Ala Leu Pro
Val Gly Ser Ser Ala Ala Val Ala Pro Leu
Gly Leu Gln Leu Met Cys Thr Ala Pro Pro
Gly Ala Val Gln Gly His Trp Ala Arg Glu
Ala Pro Gly Ala Trp Asp Cys Ser Val Glu
Asn Gly Gly Cys Glu His Ala Cys Asn Ala
Ile Pro Gly Ala Pro Arg Cys Gln Cys Pro
Ala Gly Ala Ala Leu Gln Ala Asp Gly Arg
Ser Cys Thr Ala Ser Ala Thr Gln Ser Cys
Asn Asp Leu Cys Glu His Phe Cys Val Pro
Asn Pro Asp Gln Pro Gly Ser Tyr Ser Cys
Met Cys Glu Thr Gly Tyr Arg Leu Ala Ala

Asp Gln His Arg Cys Glu Asp Val Asp Asp
Cys Ile Leu Glu Pro Ser Pro Cys Pro Gln
Arg Cys Val Asn Thr Gln Gly Gly Phe Glu
Cys His Cys Tyr Pro Asn Tyr Asp Leu Val
Asp Gly Glu Cys Val Glu Pro ... (III),

and an amino acid sequence represented by the formula (II):

Asp Ser Gly Lys Val Asp Gly Gly Asp Ser
Gly Ser Gly Glu Pro Pro Pro Ser Pro Thr
Pro Gly Ser Thr Leu Thr Pro Pro Ala Val
Gly Leu Val His Ser Gly ... (II),

attached to said amino acid sequence of formula (I) at its N-terminus and C-terminus, respectively, or a homologous variant of said polypeptide, having the activity of promoting the thrombin-catalyzed activation of protein C.

5. A process for preparing an isolated physiologically active human thrombomodulin polypeptide which has the activity of promoting the thrombin-catalyzed activation of protein C and which has a sugar chain comprising chondroitin and/or chondroitin sulfate attached to the peptide chain of said polypeptide, which comprises:
   (1) providing a human thrombomodulin composition comprising a type I thrombomodulin polypeptide and a type II thrombomodulin polypeptide,
   said type I thrombomodulin polypeptide being defined as a physiologically active human thrombomodulin polypeptide which has the activity of promoting the thrombin-catalyzed activation of protein C and which is substantially free of a sugar chain comprising chondroitin and/or chondroitin sulfate attached to the peptide chain of said polypeptide,
   said type II thrombomodulin polypeptide being defined as a physiologically active human thrombomodulin polypeptide which has the activity of promoting the thrombin-catalyzed activation of protein C and which has a sugar chain comprising chondroitin and/or chondroitin sulfate attached to the peptide chain of said polypeptide;
   (2) subjecting said human thrombomodulin composition to a separating treatment capable of separating said type I and type II thrombomodulin polypeptides from each other due to a difference in a property selected from isoelectric point, molecular weight and reactivity to an anti-chondroitin antibody and/or an anti-chondroitin sulfate antibody between said type I and type II thrombomodulin polypeptide, thereby separating said type I thrombomodulin polypeptide and said type II thrombomodulin polypeptide from each other; and
   (3) recovering said type II thrombomodulin polypeptide.

6. The process according to claim 5, wherein said property is isoelectric point and said separating treatment is ion exchange chromatography or electrophoresis.

7. The process according to claim 5, wherein said property is molecular weight and said separating treatment is gel filtration or electrophoresis.

8. The process according to claim 5, wherein said property is reactivity to an anti-chondroitin and/or an anti-chondroitin sulfate and said separating treatment is affinity chromatography.

9. A pharmaceutical composition comprising an effective amount of an isolated physiologically active human thrombomodulin polypeptide which has the activity of promoting the thrombin-catalyzed activation of protein C and which has a sugar chain comprising chondroitin and/or chondroitin sulfate attached to the peptide chain of said polypeptide, and at least one pharmaceutically acceptable carrier, diluent or excipient.

**10.** The pharmaceutical composition polypeptide according to claim 9, wherein said peptide chain comprises an amino acid sequence represented by the formula (I):

```
Val Asp Pro Cys Phe Arg Ala Asn Cys
Glu Tyr Gln Cys Gln Pro Leu Asn Gln
Thr Ser Tyr Leu Cys Val Cys Ala Glu
Gly Phe Ala Pro Ile Pro His Glu Pro
His Arg Cys Gln Met Phe Cys Asn Gln
Thr Ala Cys Pro Ala Asp Cys Asp Pro
Asn Thr Gln Ala Ser Cys Glu Cys Pro
Glu Gly Tyr Ile Leu Asp Asp Gly Phe
Ile Cys Thr Asp Ile Asp Glu Cys Glu
Asn Gly Gly Phe Cys Ser Gly Val Cys
His Asn Leu Pro Gly Thr Phe Glu Cys
Ile Cys Gly Pro Asp Ser Ala Leu Val
Arg His Ile Gly Thr Asp Cys  ... (I)
```

**11.** The pharcaceutical composition according to claim 10, wherein said peptide chain further comprises an amino acid sequence represented by the formula (II):

```
Asp Ser Gly Lys Val Asp Gly Gly Asp Ser
Gly Ser Gly Glu Pro Pro Pro Ser Pro Thr
Pro Gly Ser Thr Leu Thr Pro Pro Ala Val
Gly Leu Val His Ser Gly ... (II).
```

attached to said amino acid sequence of formula (I) at its C-terminus.

**12.** The pharmaceutical comosition according to claim 10, wherein said peptide chain further comprises an amino acid sequence represented by the formula (III):

Ala Pro Ala Glu Pro Gln Pro Gly Gly Ser
Gln Cys Val Glu His Asp Cys Phe Ala Leu
Tyr Pro Gly Pro Ala Thr Phe Leu Asn Ala
Ser Gln Ile Cys Asp Gly Leu Arg Gly His
Leu Met Thr Val Arg Ser Ser Val Ala Ala
Asp Val Ile Ser Leu Leu Leu Asn Gly Asp
Gly Gly Val Gly Arg Arg Arg Leu Trp Ile
Gly Leu Gln Leu Pro Pro Gly Cys Gly Asp
Pro Lys Arg Leu Gly Pro Leu Arg Gly Phe
Gln Trp Val Thr Gly Asp Asn Asn Thr Ser
Tyr Ser Arg Trp Ala Arg Leu Asp Leu Asn
Gly Ala Pro Leu Cys Gly Pro Leu Cys Val
Ala Val Ser Ala Ala Glu Ala Thr Val Pro
Ser Glu Pro Ile Trp Glu Glu Gln Gln Cys
Glu Val Lys Ala Asp Gly Phe Leu Cys Glu
Phe His Phe Pro Ala Thr Cys Arg Pro Leu
Ala Val Glu Pro Gly Ala Ala Ala Ala Ala
Val Ser Ile Thr Tyr Gly Thr Pro Phe Ala
Ala Arg Gly Ala Asp Phe Gln Ala Leu Pro
Val Gly Ser Ser Ala Ala Val Ala Pro Leu

Gly Leu Gln Leu Met Cys Thr Ala Pro Pro
Gly Ala Val Gln Gly His Trp Ala Arg Glu
Ala Pro Gly Ala Trp Asp Cys Ser Val Glu
Asn Gly Gly Cys Glu His Ala Cys Asn Ala
Ile Pro Gly Ala Pro Arg Cys Gln Cys Pro
Ala Gly Ala Ala Leu Gln Ala Asp Gly Arg
Ser Cys Thr Ala Ser Ala Thr Gln Ser Cys
Asn Asp Leu Cys Glu His Phe Cys Val Pro
Asn Pro Asp Gln Pro Gly Ser Tyr Ser Cys
Met Cys Glu Thr Gly Tyr Arg Leu Ala Ala
Asp Gln His Arg Cys Glu Asp Val Asp Asp
Cys Ile Leu Glu Pro Ser Pro Cys Pro Gln
Arg Cys Val Asn Thr Gln Gly Gly Phe Glu
Cys His Cys Tyr Pro Asn Tyr Asp Leu Val
Asp Gly Glu Cys Val Glu Pro ... (III),

and an amino acid sequence represented by the formula (II):

Asp Ser Gly Lys Val Asp Gly Gly Asp Ser
Gly Ser Gly Glu Pro Pro Pro Ser Pro Thr
Pro Gly Ser Thr Leu Thr Pro Pro Ala Val
Gly Leu Val His Ser Gly ... (II),

attached to said amino acid sequence of formula (I) at its N-terminus and C-terminus, respectively.

# FIG. 1 (a)

Signal peptide portion (residues 1–20)

```
  1                                                                              20
Met Leu Gly Val Leu Val Leu Gly Ala Leu Ala Leu Ala Gly Leu Gly Phe Pro Ala Pro
 21                                                                              40
Ala Glu Pro Gln Pro Gly Gly Ser Gln Cys Val Glu His Asp Cys Phe Ala Leu Tyr Pro
 41                                                                              60
Gly Pro Ala Thr Phe Leu Asn Ala Ser Gln Ile Cys Asp Gly Leu Arg Gly His Leu Met
 61                                                                              80
Thr Val Arg Ser Ser Val Ala Ala Asp Val Ile Ser Leu Leu Leu Asn Gly Asp Gly Gly
 81                                                                             100
Val Gly Arg Arg Arg Leu Trp Ile Gly Leu Gln Leu Pro Pro Gly Cys Gly Asp Pro Lys
101                                                                             120
Arg Leu Gly Pro Leu Arg Gly Phe Gln Trp Val Thr Gly Asp Asn Asn Thr Ser Tyr Ser
121                                                                             140
Arg Trp Ala Arg Leu Asp Leu Asn Gly Ala Pro Leu Cys Gly Pro Leu Cys Val Ala Val
141                                                                             160
Ser Ala Ala Glu Ala Thr Val Pro Ser Glu Pro Ile Trp Glu Glu Gln Gln Cys Glu Val
161                                                                             180
Lys Ala Asp Gly Phe Leu Cys Glu Phe His Phe Pro Ala Thr Cys Arg Pro Leu Ala Val
```

# FIG. 1 (b)

181 200
Glu Pro Gly Ala Ala Ala Ala Ala Val Ser Ile Thr Tyr Gly Thr Pro Phe Ala Ala Arg
201 220
Gly Ala Asp Phe Gln Ala Leu Pro Val Gly Ser Ser Ala Ala Val Ala Pro Leu Gly Leu
221 240
Gln Leu Met Cys Thr Ala Pro Pro Gly Ala Val Gln Gly His Trp Ala Arg Glu Ala Pro
241 260
Gly Ala Trp Asp Cys Ser Val Glu Asn Gly Gly Cys Glu His Ala Cys Asn Ala Ile Pro
261 280
Gly Ala Pro Arg Cys Gln Cys Pro Ala Gly Ala Ala Leu Gln Ala Asp Gly Arg Ser Cys
281 300
Thr Ala Ser Ala Thr Gln Ser Cys Asn Asp Leu Cys Glu His Phe Cys Val Pro Asn Pro
301 320
Asp Gln Pro Gly Ser Tyr Ser Cys Met Cys Glu Thr Gly Tyr Arg Leu Ala Ala Asp Gln
321 340
His Arg Cys Glu Asp Val Asp Asp Cys Ile Leu Glu Pro Ser Pro Cys Pro Gln Arg Cys
341 360
Val Asn Thr Gln Gly Gly Phe Glu Cys His Cys Tyr Pro Asn Tyr Asp Leu Val Asp Gly
361 380
Glu Cys Val Glu Pro Val Asp Pro Cys Phe Arg Ala Asn Cys Glu Tyr Gln Cys Gln Pro

# FIG. 1 (c)

381 400
Leu Asn Gln Thr Ser Tyr Leu Cys Val Cys Ala Glu Gly Phe Ala Pro Ile Pro His Glu
401 420
Pro His Arg Cys Gln Met Phe Cys Asn Gln Thr Ala Cys Pro Ala Asp Cys Asp Pro Asn
421 440
Thr Gln Ala Ser Cys Glu Cys Pro Glu Gly Tyr Ile Leu Asp Asp Gly Phe Ile Cys Thr
441 460
Asp Ile Asp Glu Cys Glu Asn Gly Gly Phe Cys Ser Gly Val Cys His Asn Leu Pro Gly
461 480
Thr Phe Glu Cys Ile Cys Gly Pro Asp Ser Ala Leu Val Arg His Ile Gly Thr Asp Cys
481 500
Asp Ser Gly Lys Val Asp Gly Gly Asp Ser Gly Ser Gly Glu Pro Pro Pro Ser Pro Thr
501 520
Pro Gly Ser Thr Leu Thr Pro Pro Ala Val Gly Leu Val His Ser Gly Leu Leu Ile Gly
521 540
Ile Ser Ile Ala Ser Leu Cys Leu Val Val Ala Leu Leu Ala Leu Leu Cys His Leu Arg
541 560
Lys Lys Gln Gly Ala Ala Arg Ala Lys Met Glu Tyr Lys Cys Ala Ala Pro Ser Lys Glu
561
Val Val Leu Gln His Val Arg Thr Glu Arg Thr Pro Gln Arg Leu

FIG. 2

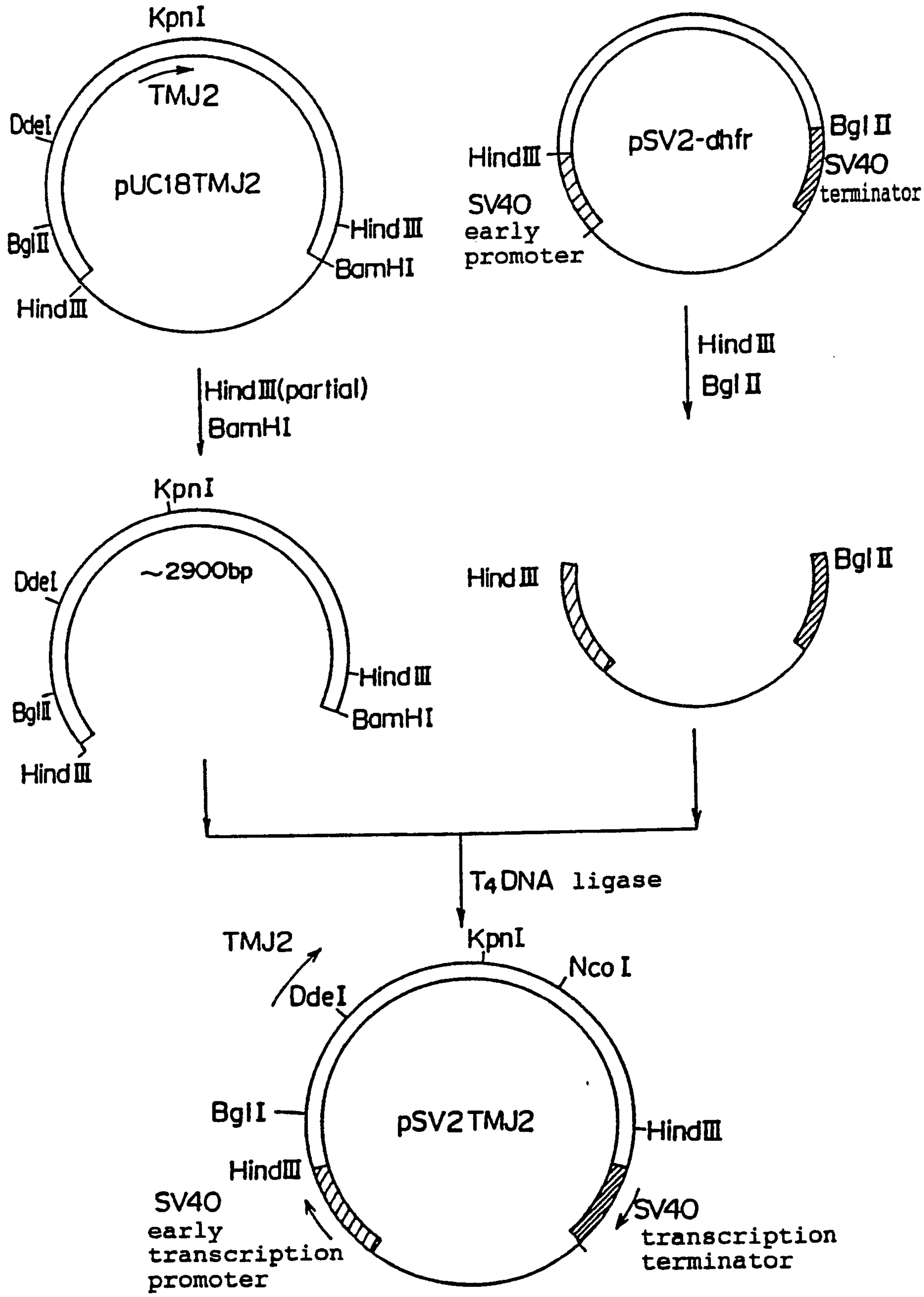
KpnI
TMJ2
DdeI
pUC18TMJ2
BglII
HindIII
HindIII
BamHI
pSV2-dhfr
HindIII
SV40
early
promoter
BglII
SV40
terminator
HindIII(partial)
BamHI
HindIII
BglII
KpnI
~2900bp
DdeI
BglII
HindIII
HindIII
BamHI
HindIII
BglII
T4DNA ligase
TMJ2
KpnI
NcoI
DdeI
BglI
pSV2TMJ2
HindIII
HindIII
SV40
early
transcription
promoter
SV40
transcription
terminator

FIG. 3

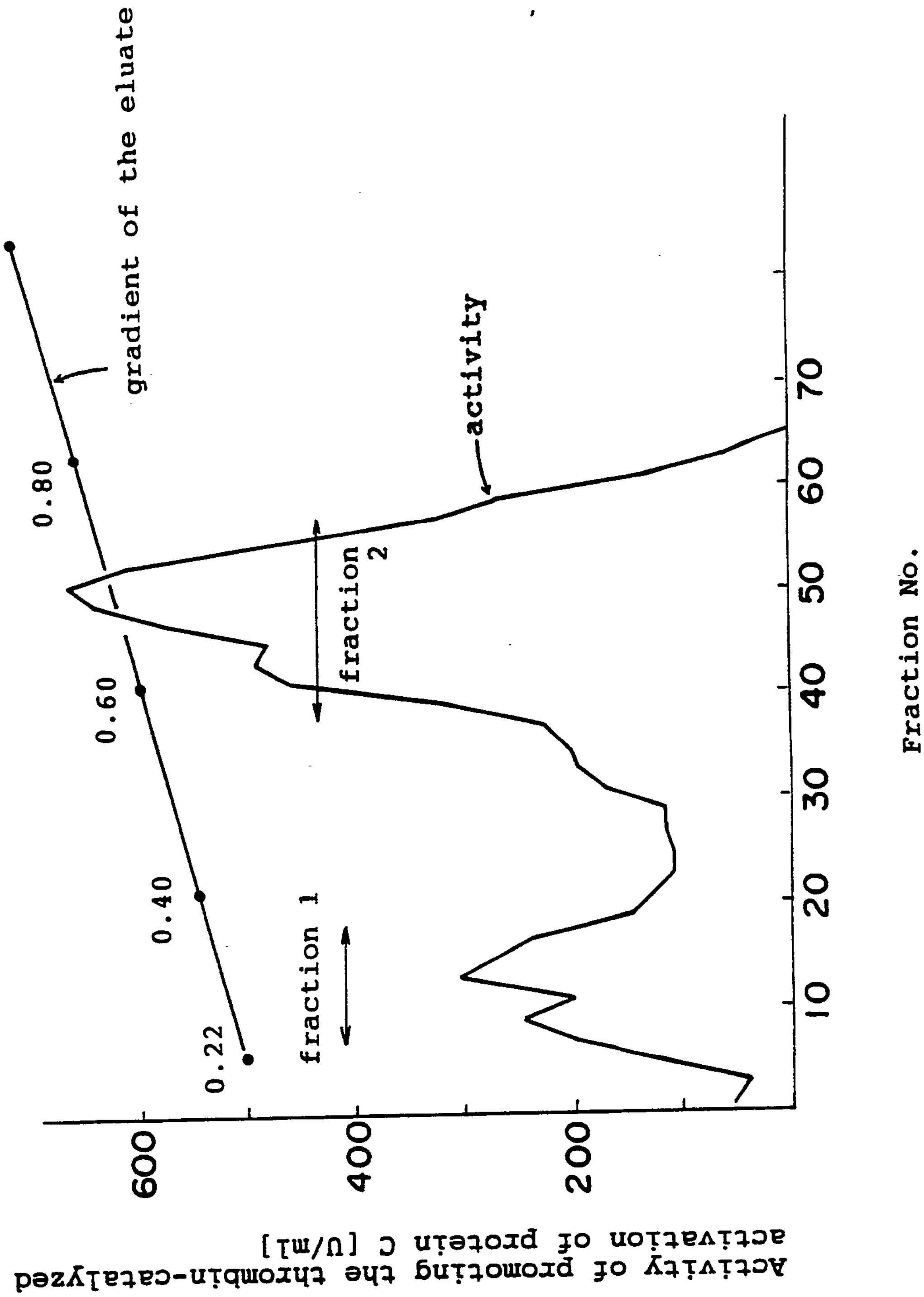
gradient of the eluate
1.00M[NaCl]
0.80
0.60
0.40
0.22
activity
fraction 1
fraction 2
600
400
200
10
20
30
40
50
60
70
Fraction No.
Activity of promoting the thrombin-catalyzed activation of protein C [U/ml]

FIG.4

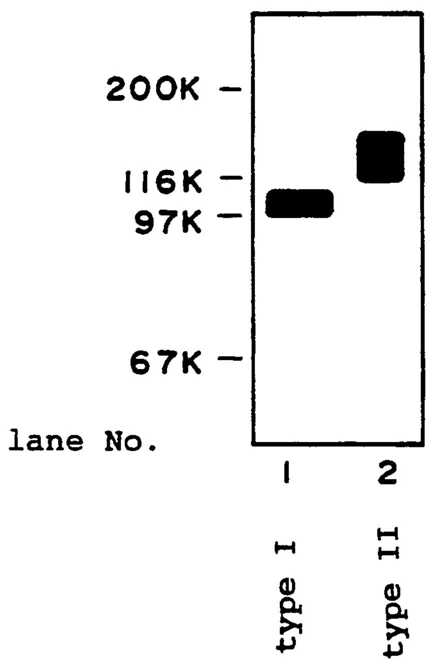

# FIG.5

```
              1                                    10
Type I   :  Phe Pro Ala Pro Ala Glu Pro Gln Pro Gly
                  1                                    10
                 Ala Pro Ala Glu Pro Gln Pro Gly Gly Ser

              1                                    10
Type II  :  Phe Pro Ala Pro Ala Glu Pro Gln Pro Gly Gly Ser Gln Cys Val
                  1                                    10
                 Ala Pro Ala Glu Pro Gln Pro Gly Gly Ser Gln Cys Val Glu His
```

FIG.6

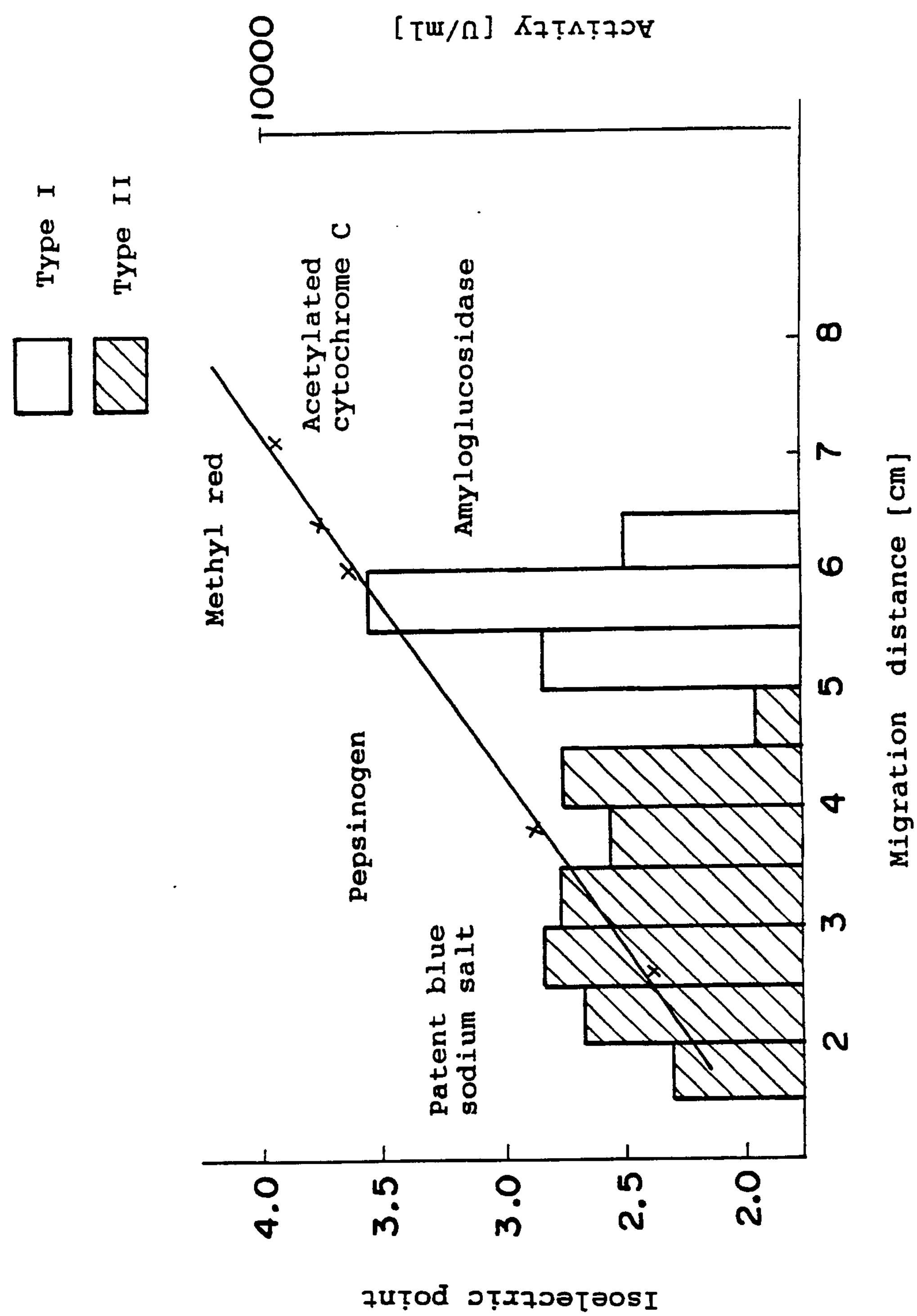
Type I
Type II
10000
Activity [U/ml]
Acetylated cytochrome C
Amyloglucosidase
Methyl red
Pepsinogen
Patent blue sodium salt
4.0
3.5
3.0
2.5
2.0
Isoelectric point
2
3
4
5
6
7
8
Migration distance [cm]

FIG.7

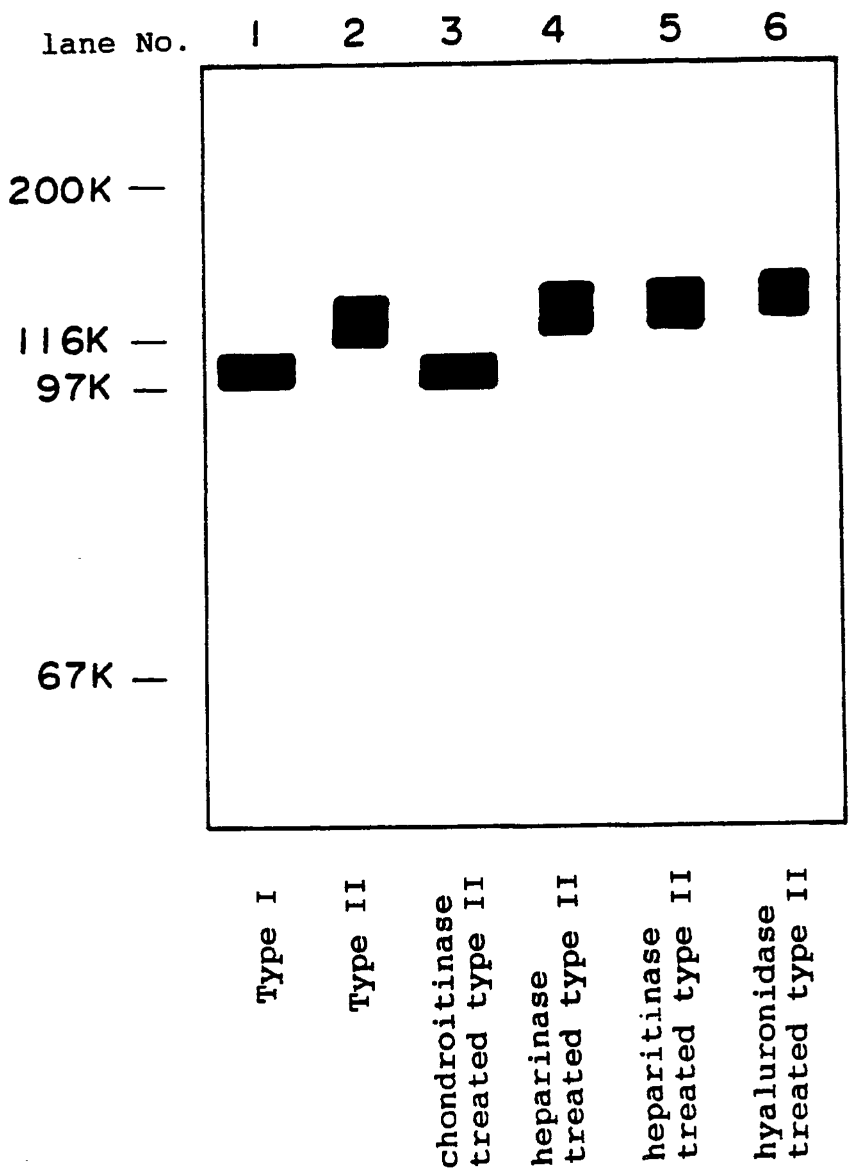
lane No. 1 2 3 4 5 6
200K
116K
97K
67K
Type I
Type II
chondroitinase treated type II
heparinase treated type II
heparitinase treated type II
hyaluronidase treated type II

FIG. 8

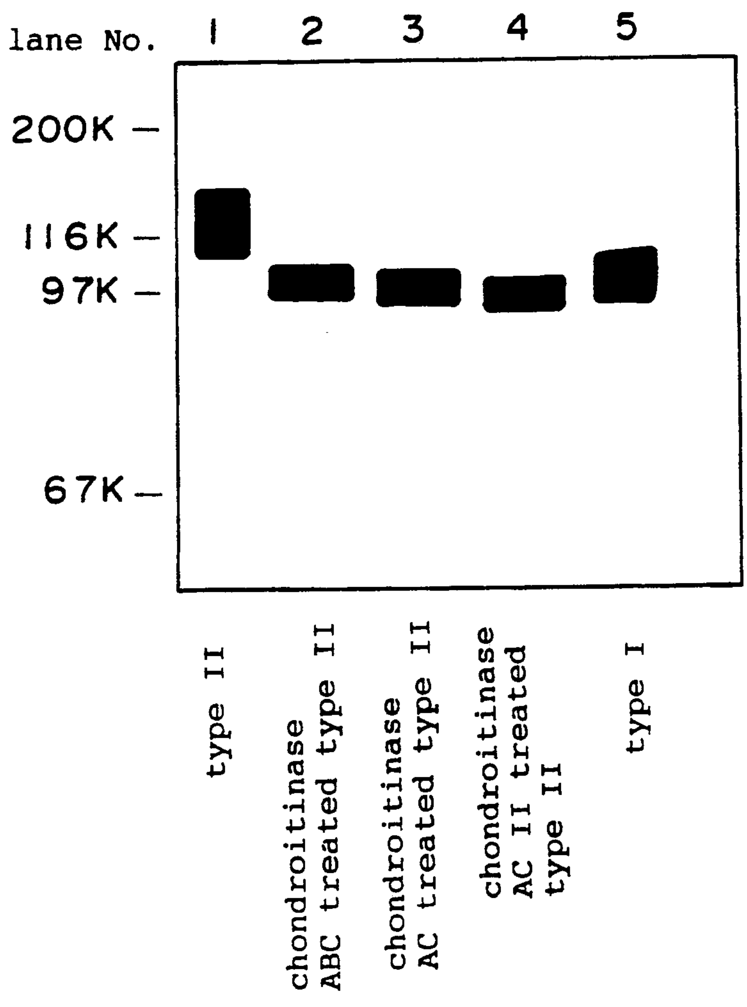
lane No.
1
2
3
4
5
200K
116K
97K
67K
type II
chondroitinase ABC treated type II
chondroitinase AC treated type II
chondroitinase AC II treated type II
type I

# FIG. 9

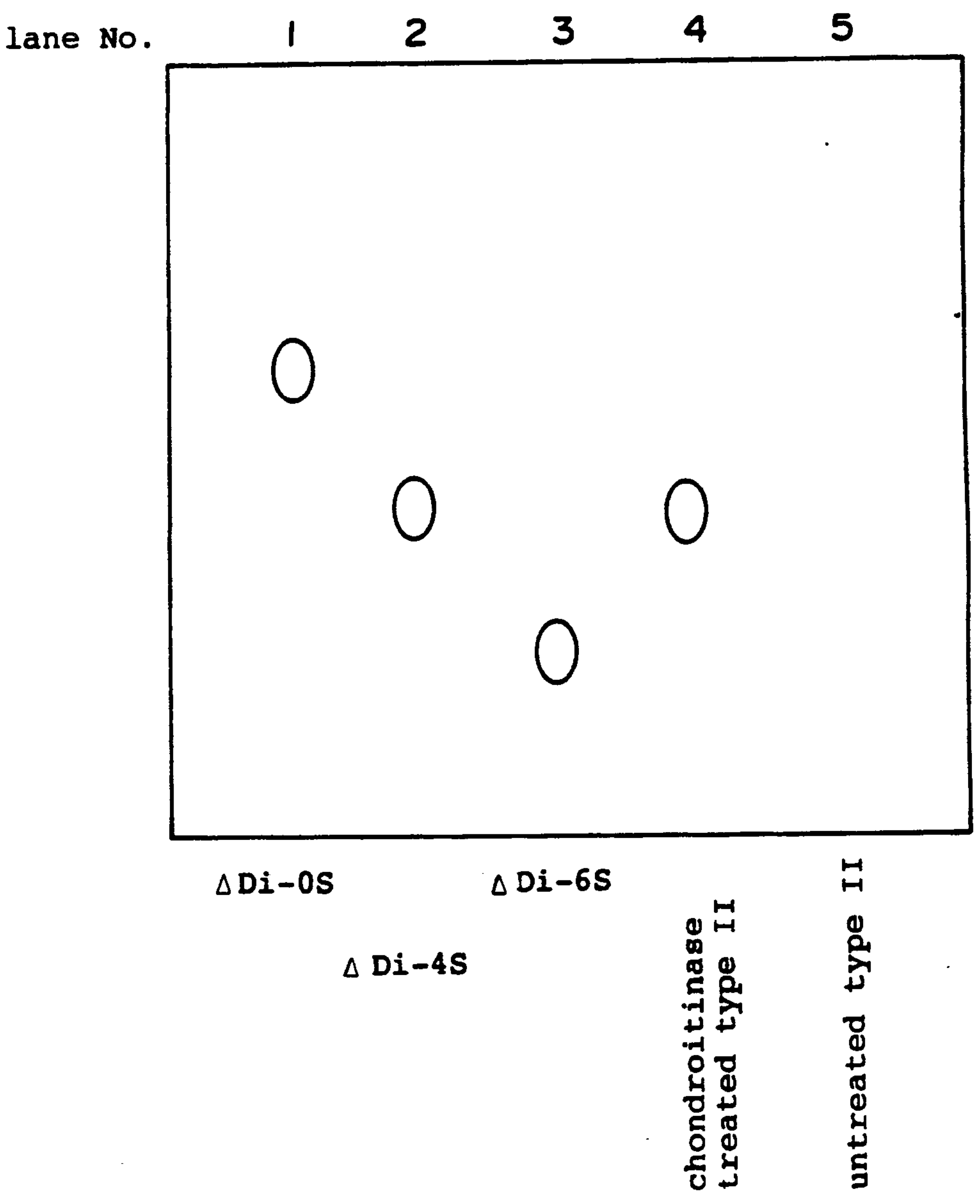
lane No.
1
2
3
4
5
Δ Di-0S
Δ Di-4S
Δ Di-6S
chondroitinase treated type II
untreated type II

FIG.10

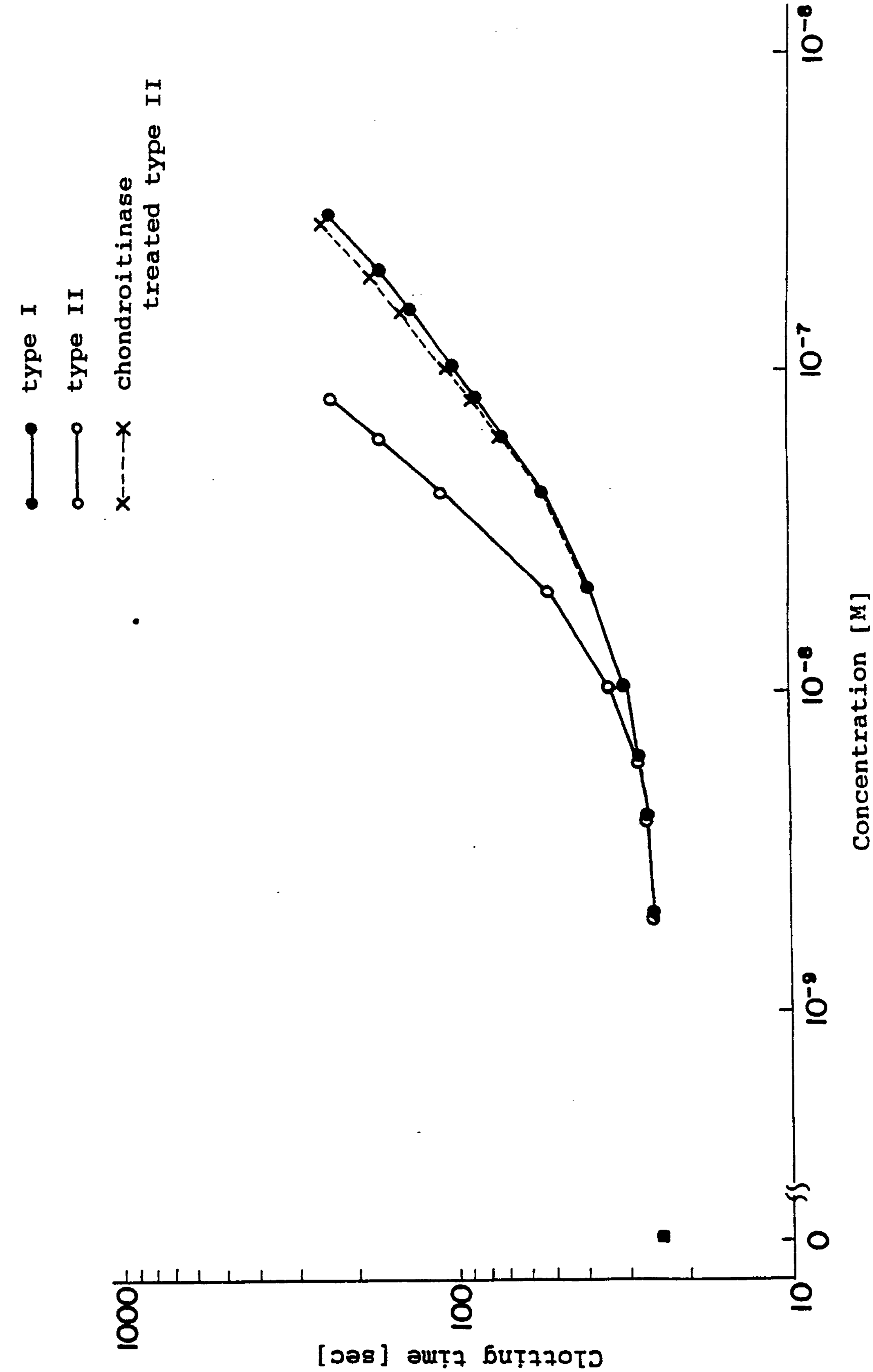
type I
type II
chondroitinase treated type II
Clotting time [sec]
1000
100
10
0
$10^{-9}$
$10^{-8}$
$10^{-7}$
$10^{-6}$
Concentration [M]

FIG.11

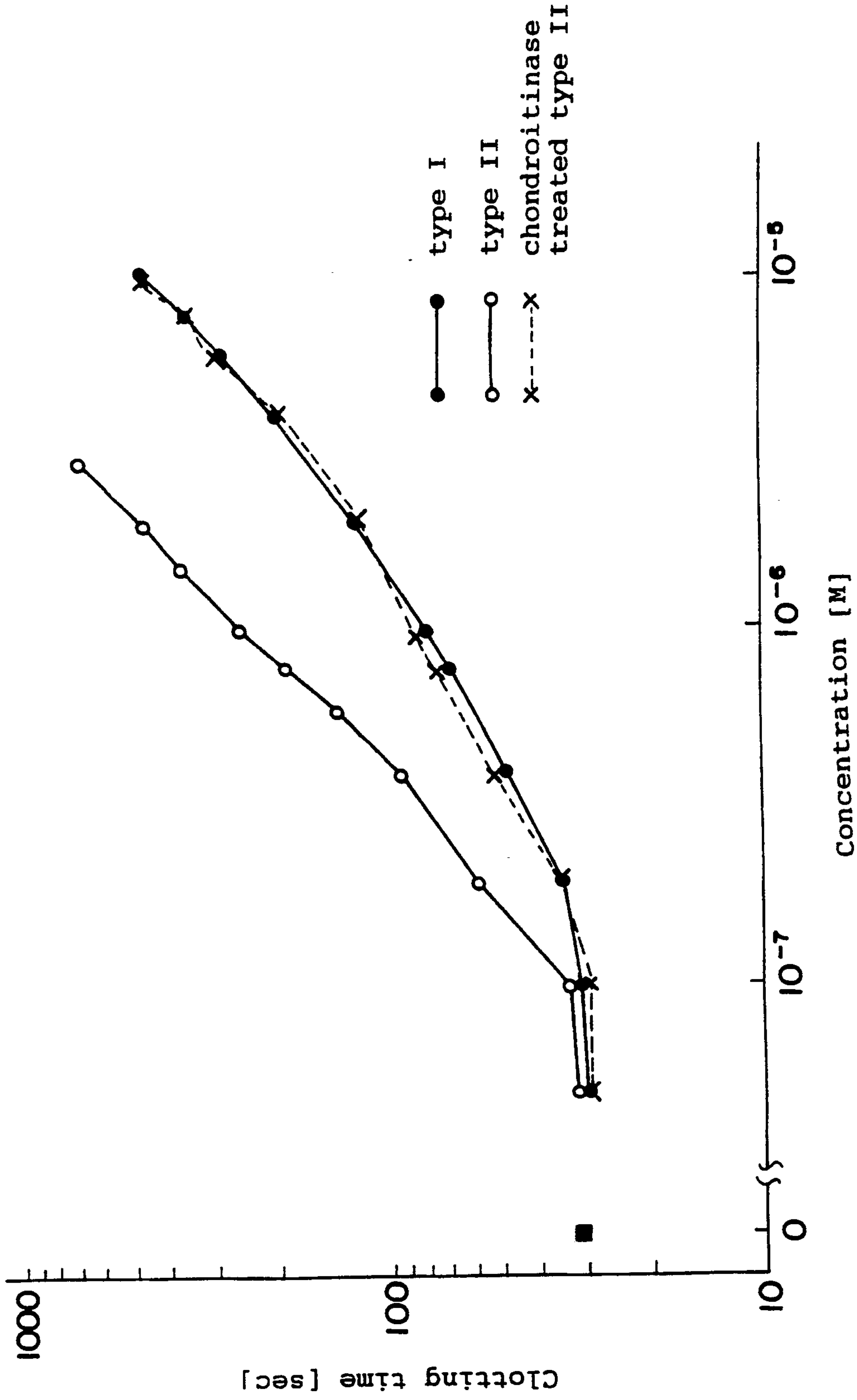
type I
type II
chondroitinase treated type II
Clotting time [sec]
1000
100
10
0
$10^{-7}$
$10^{-6}$
$10^{-5}$
Concentration [M]

FIG.12

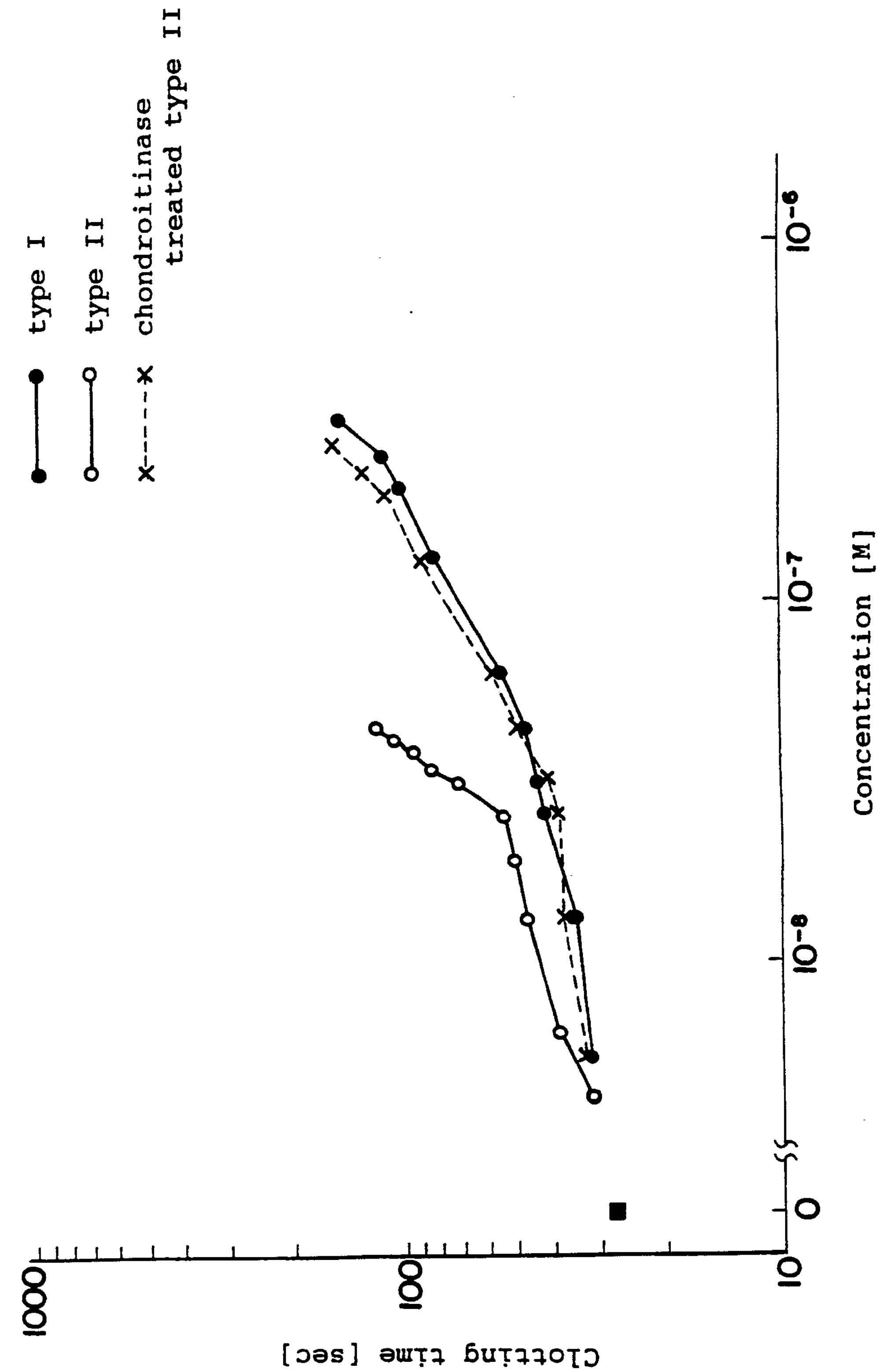

type I
type II
chondroitinase treated type II
Clotting time [sec]
1000
100
10
0
$10^{-8}$
$10^{-7}$
$10^{-6}$
Concentration [M]

# FIG.13

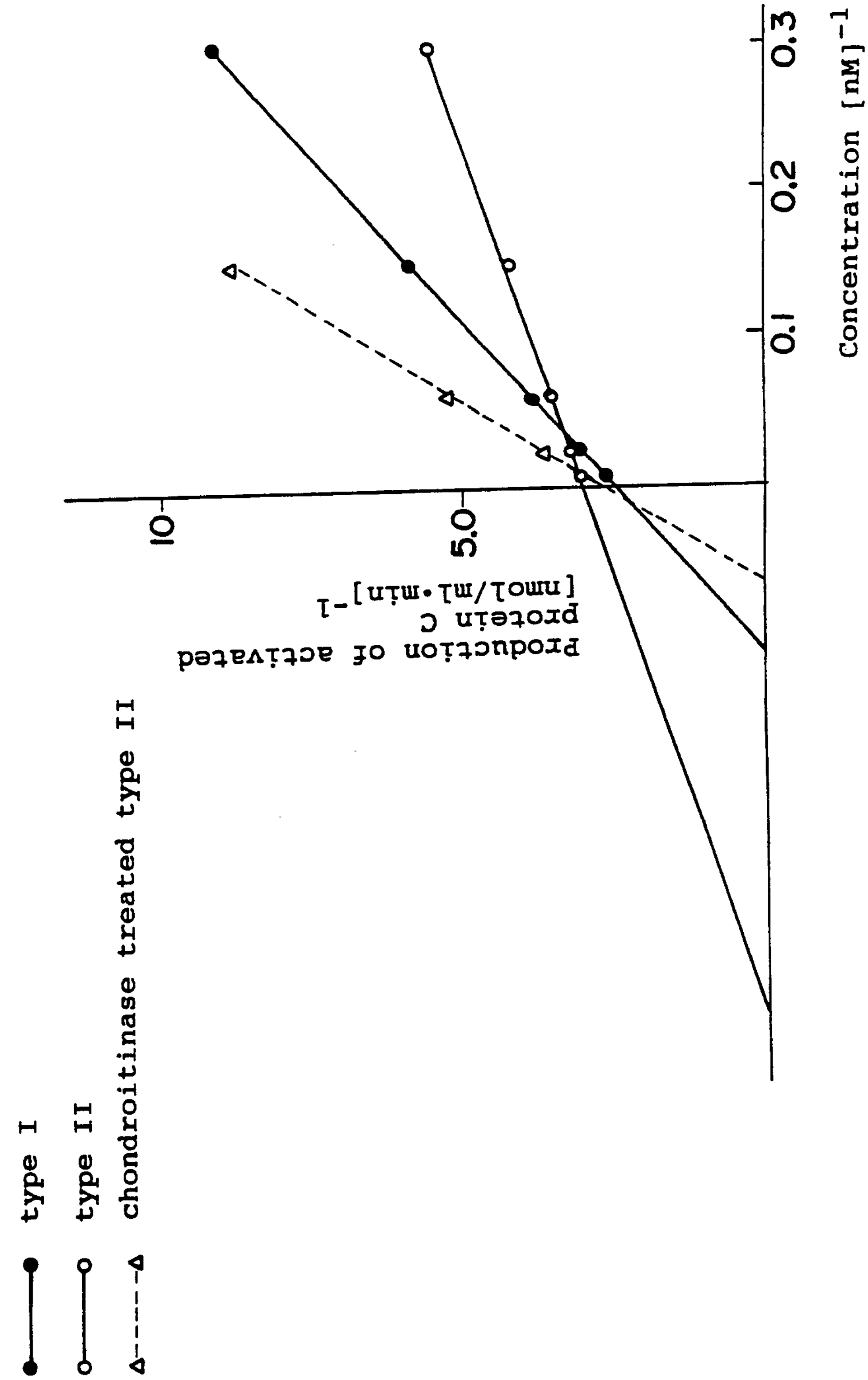
type I
type II
chondroitinase treated type II
Production of activated protein C [nmol/ml·min]-1
10
5.0
0.1
0.2
0.3
Concentration [nM]-1

FIG.14

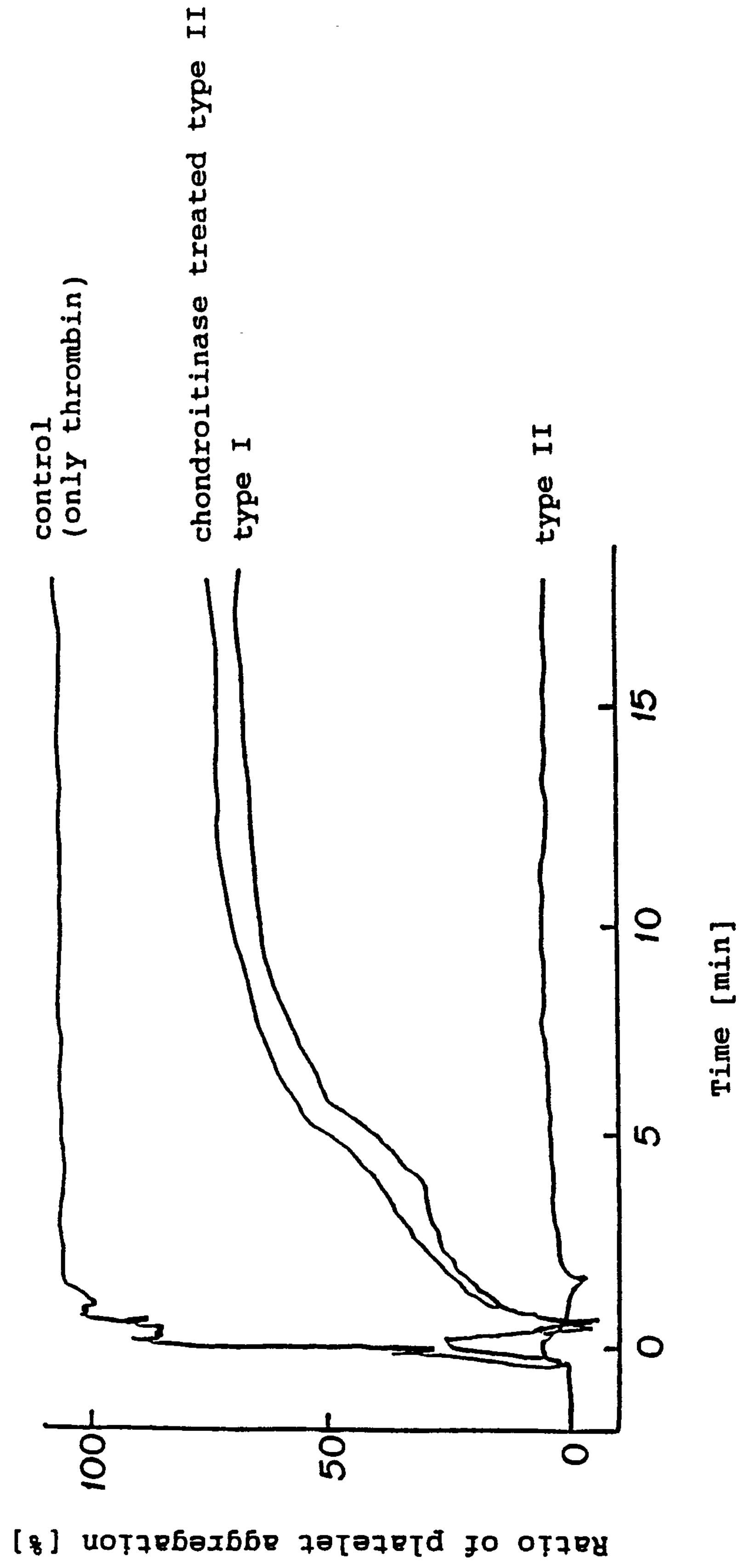
control
(only thrombin)
chondroitinase treated type II
type I
type II
Time [min]
Ratio of platelet aggregation [%]
0
50
100
0
5
10
15

FIG.15

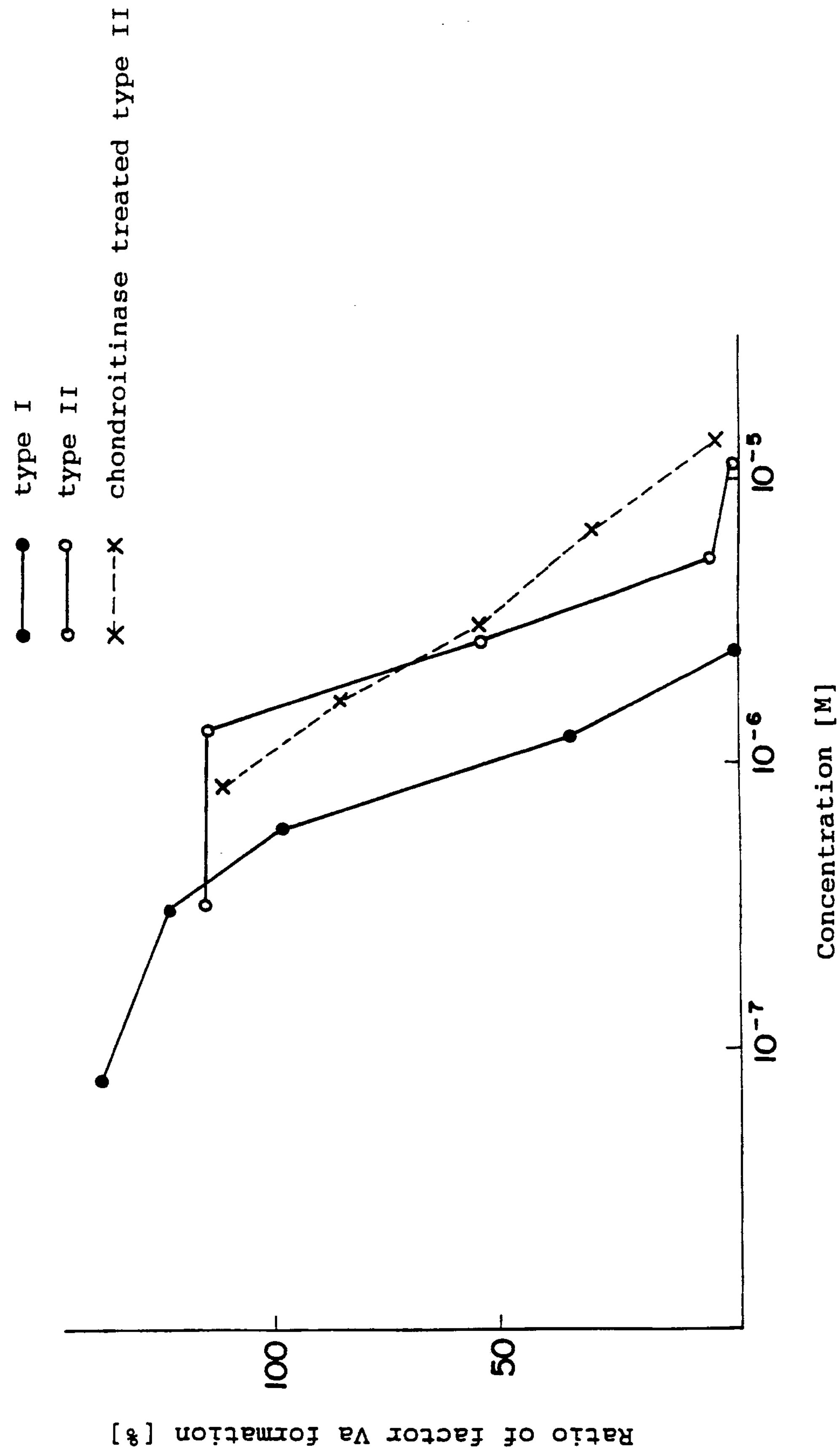
type I
type II
chondroitinase treated type II
100
50
10⁻⁷
10⁻⁶
10⁻⁵
Concentration [M]
Ratio of factor Va formation [%]

# FIG. 16

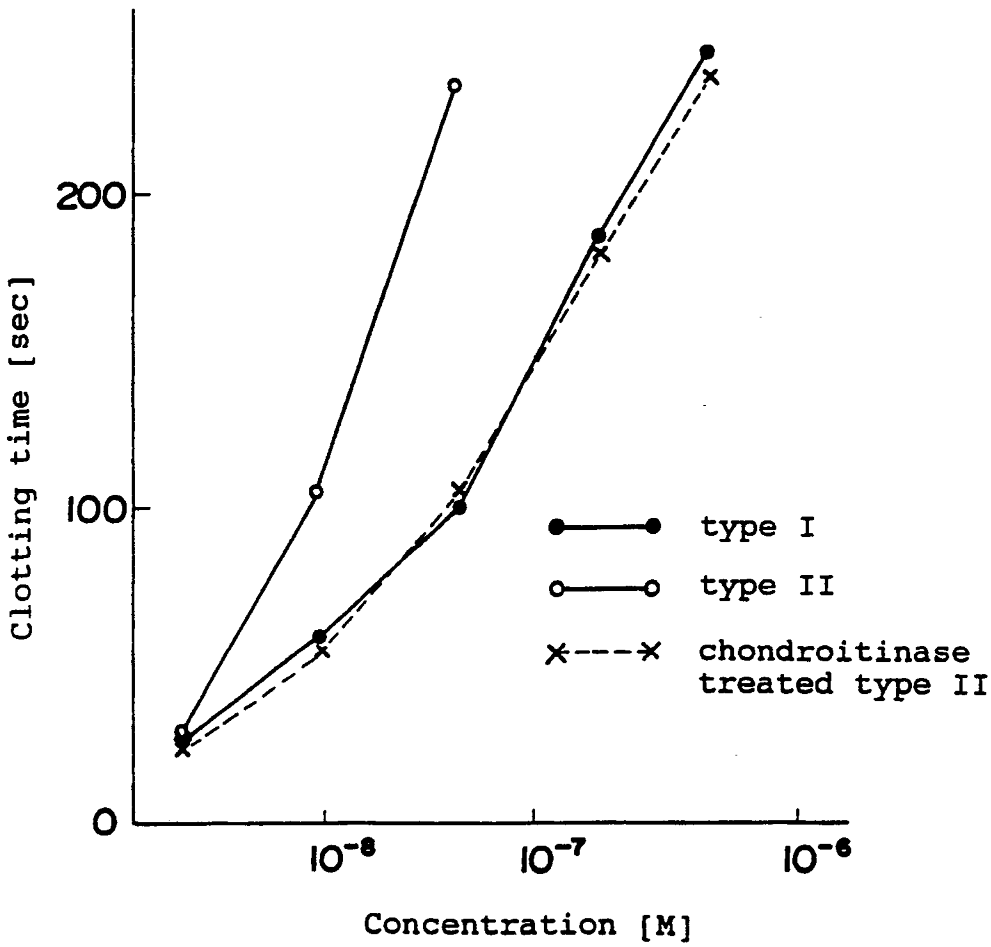

Clotting time [sec]
200
100
0
10$^{-8}$
10$^{-7}$
10$^{-6}$
Concentration [M]
type I
type II
chondroitinase treated type II

FIG. 17

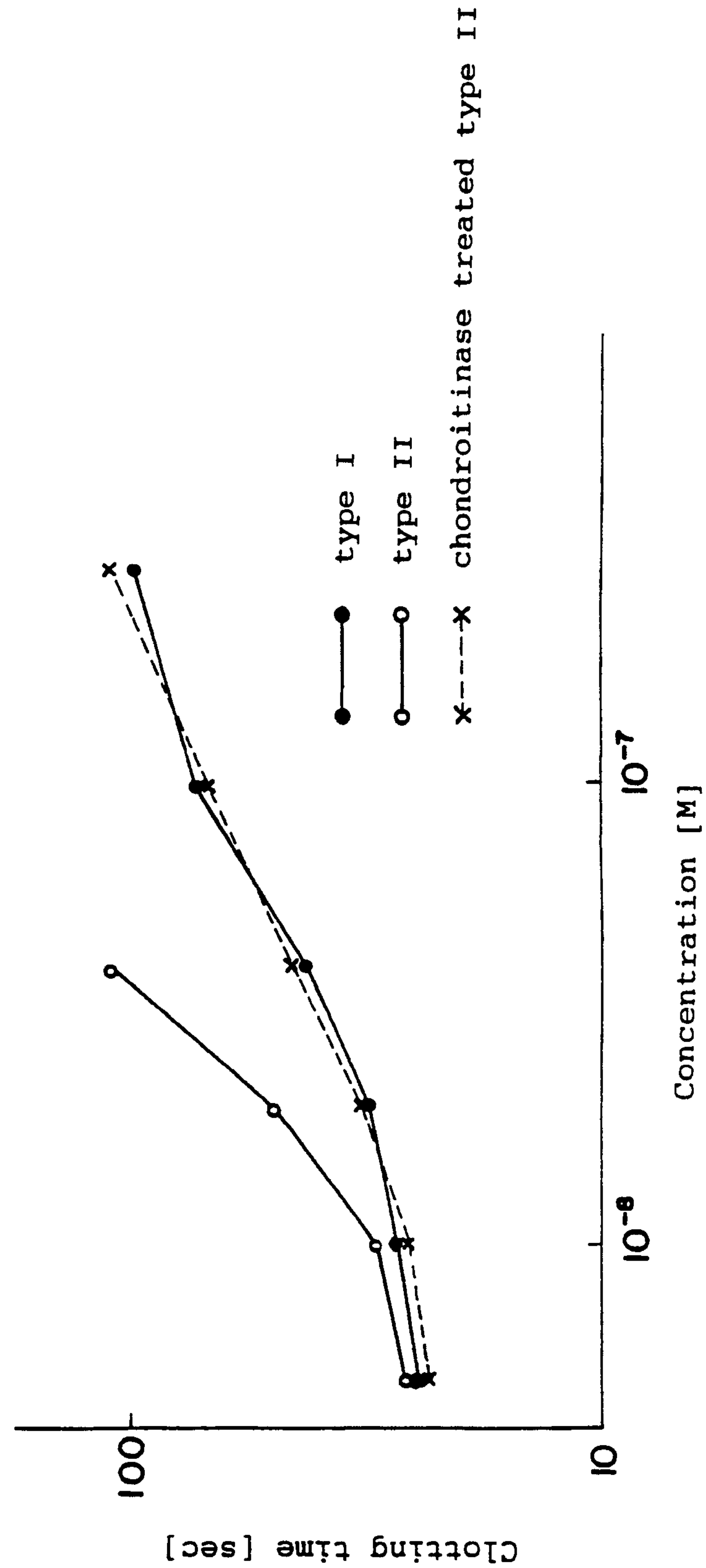
type I
type II
chondroitinase treated type II
100
10
Clotting time [sec]
$10^{-8}$
$10^{-7}$
Concentration [M]

FIG. 18

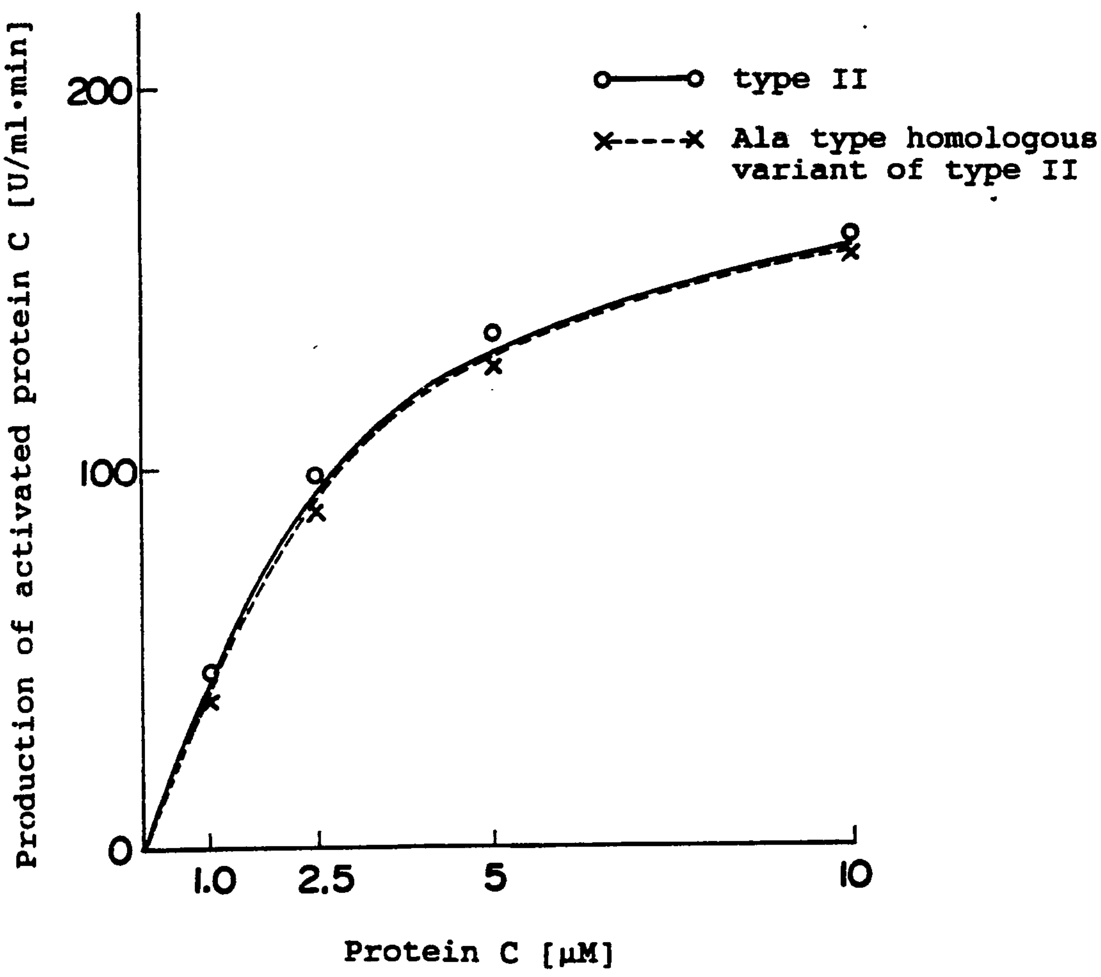
Production of activated protein C [U/ml·min]
200
100
0
type II
Ala type homologous variant of type II
1.0
2.5
5
10
Protein C [μM]

# INTERNATIONAL SEARCH REPORT

International Application No PCT/JP90/01234

**I. CLASSIFICATION OF SUBJECT MATTER** (if several classification symbols apply, indicate all) [6]

According to International Patent Classification (IPC) or to both National Classification and IPC

Int. $Cl^5$ C07K15/14, C12P21/00, A61K37/02//C12N15/12,
(C12P21/00, C12R1:91)

**II. FIELDS SEARCHED**

Minimum Documentation Searched [7]

| Classification System | Classification Symbols |
|---|---|
| IPC | C07K15/06, 15/14, C12P21/00-21/02, A61K37/02, C12N15/12 |

Documentation Searched other than Minimum Documentation to the Extent that such Documents are Included in the Fields Searched [8]

Biological Abstracts Data Base (BIOSIS)

**III. DOCUMENTS CONSIDERED TO BE RELEVANT** [9]

| Category [*] | Citation of Document, [11] with indication, where appropriate, of the relevant passages [12] | Relevant to Claim No. [13] |
|---|---|---|
| A | WO, A1, 87/00050 (Asahi Chemical Industry Co., Ltd.), January 15, 1987 (15. 01. 87) & EP, A2, 239644 | 1-12 |
| A | JP, A, 63-301791 (Washington University), December 8, 1988 (08. 12. 88) & EP, A2, 290419 & US, A, 4912207 | 1-12 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| December 13, 1990 (13. 12. 90) | December 25, 1990 (25. 12. 90) |
| **International Searching Authority** | **Signature of Authorized Officer** |
| Japanese Patent Office | |